# EUROPEAN PATENT SPECIFICATION

(11) **EP 3 963 330 B1**
(45) Date of publication and mention of the grant of the patent: **28.01.2026**
(21) Application number: 20721270.5
(22) Date of filing: 01.05.2020
(51) Int. Cl.: G01N 33/574

(54) **METHOD FOR THE DETECTION OF PROSTATE CANCER**
VERFAHREN FÜR DEN NACHWEIS VON PROSTATAKREBS
PROCÉDÉ DE DÉTECTION DU CANCER DE LA PROSTATE

(30) Priority: 02.05.2019 GB 201906201
(43) Date of publication of application: 09.03.2022
(73) Proprietor: Belgian Volition SRL, 5032 Isnes (BE)
(72) Inventor: MICALLEF, Jacob Vincent, 5032 Isnes (BE); ECCLESTON, Mark Edward, 5032 Isnes (BE); HERZOG, Marielle, 5032 Isnes (BE); TERRELL, Jason Bradley, 5032 Isnes (BE)
(74) Representative: Sagittarius IP
(86) International application number: PCT/EP2020/062191
(87) International publication number: WO 2020/221922

(56) References cited:
- WO-A1-99/49083
- JASPREET SINGH BATRA ET AL: "A Quest to Identify Prostate Cancer Circulating Biomarkers with a Bench-to-Bedside Potential", JOURNAL OF BIOMARKERS, vol. 2014, 1 January 2014 (2014-01-01), pages 1 - 12, XP055655042, ISSN: 2090-8660, DOI: 10.1155/2014/321680
- NOBUKAZU KOMATSU ET AL: "Gene expression profiles in peripheral blood as a biomarker in cancer patients receiving peptide vaccination", CANCER, vol. 118, no. 12, 15 June 2012 (2012-06-15), pages 3208 - 3221, XP055083382, ISSN: 0008-543X, DOI: 10.1002/cncr.26636

## Description

### FIELD OF THE INVENTION

The invention relates to a body fluid test method for the detection of prostate cancer, in particular aggressive prostate cancer. It also relates to monitoring of patients with low risk prostate tumours and selection of patients for prostate biopsy as well as selection of patients for prostate surgery or therapy.

### BACKGROUND OF THE INVENTION

Prostate cancer (PCa) is a common disease in men with a high mortality rate. PCa is the second leading cause of cancer death among men in the USA after lung cancer and the fifth leading cause world-wide.

Prostate cancer is often discovered by a positive PSA (Prostate Specific Antigen) blood test or a DRE (Digital Rectal Examination) in which the doctor inserts a gloved, lubricated finger into the rectum to feel the surface of the prostate for any irregularities. A high blood level of PSA (typically >3ng/ml) and/or an abnormal DRE result are reasons to suspect PCa, but PCa is also often discovered by various non-specific symptoms or other signs which may include difficulty in urination, frequent urination, painful urination, blood in the urine, blood in the seminal fluid, erectile dysfunction and discomfort when sitting. If the cancer has become metastatic symptoms may also include bone pain, swelling of the lower leg, weight loss or fatigue.

Men with suspected PCa will usually be referred for prostate biopsy to confirm the diagnosis by identifying abnormal cancer cells in the prostate tissue. Prostate biopsy is a highly invasive procedure involving insertion of a needle into the prostate gland through the wall of the rectum. The biopsy needle is inserted multiple times to remove up to 20 small samples of tissue for pathologic analysis, typically guided using transrectal ultrasound (TRUS). Prostate biopsy carries risks including rectal bleeding at the biopsy site, blood in the semen, blood in the urine, difficulty in urinating and infection. Due to the fairly poor discrimination of first line tests, most men referred have a negative biopsy result with no cancer cells found.

A positive prostate biopsy result, in which cancer tissue is found, is usually expressed in terms of the Gleason grading system where the cancer tissue is graded on a scale from 1-5. Cancers with a Gleason score of 7 (3+4) or below are considered low grade cancers. Men with low grade PCa are generally not treated for cancer but are monitored to check that the cancer does not progress. Cancers with a Gleason score of 7 (4+3) or above are considered high grade cancers. Men with high grade cancers are generally treated.

The Gleason score is used by clinicians in combination with PSA levels, DRE results and tumour TNM classification as well as other factors, to categorise patients as low risk, intermediate risk or high risk clinically to determine whether they should be treated for PCa or monitored. The TNM classification involves the size and degree of spread of the tumour (T), whether the tumour has spread to the lymph nodes and how many nodes (N) and whether the cancer has metastasized to other parts of the body (M).

The risk category for a patient is based on the serum PSA level as well as the Gleason Score and the clinical size and extent of the disease. Prostate cancer can be separated into various categories of increasing clinical risk from low risk to intermediate risk and high risk according to the risk stratification system used by the Memorial Sloan-Kettering and Seattle groups (Humphrey; 2014 and Barry and Nelson; 2015). Men with low risk prostate cancer are generally not treated for cancer but are monitored to check that the cancer remains low risk. High risk cancers are considered aggressive and potentially lethal, therefore men with high risk cancers are generally treated. Intermediate levels of treatment and/or monitoring are appropriate for men with cancers of intermediate risk.

Clinical risk stratification may be further refined, for example by dividing the intermediate risk group into an intermediate favourable risk group and an intermediate unfavourable risk group (NCCN; 2018). Intermediate risk category patients may be treated depending on other factors and after discussion with the patient.

Treatments available for PCa include surgery, radiotherapy including brachytherapy, hormone therapy including surgical and medical castration as well as a variety of drug treatments. Treatment may result in side effects which can have severe consequences for quality of life. Treatment should not be administered inappropriately to men with low grade PCa disease. To avoid inappropriate treatment, men with low grade disease may be monitored for years by watchful waiting (long term monitoring of low grade PCa to avoid treatment unless symptoms appear) or by active surveillance (monitoring by regular hospital tests including repeat prostate biopsies and magnetic resonance imaging (MRI) scans). For the majority of men in whom there is no disease progression repeat biopsies represent an unnecessary invasive intervention.

Most men with elevated PSA levels do not have high grade PCa and the majority of men referred for prostate biopsy have either no cancer or low grade indolent cancers which are slow growing and not life threatening during the course of the patient's natural lifetime, particularly if diagnosed in elderly men. Studies have shown that, whilst screening men with a PSA test does increase the frequency of PCa diagnosis, it does not reduce mortality. A recent study of 419,582 men aged 50-69 years conducted in the United Kingdom investigated the outcome over 10 years of screening men for PCA with a PSA test. Screening resulted in an increase in PCa diagnosis from 3.6% of men in the unscreened group to 4.3% in the screened group, but more of the screened cancers diagnosed were low grade with a Gleason score of 6 or lower. Despite the increase in PCa diagnosis, the PCa mortality rate was relatively unaffected at 0.30 deaths per 1000 person-years in the screened group compared to 0.31 deaths per 1000 person-years in the unscreened group (Martin et al; 2018). This means that many extra men were subjected to invasive biopsies and other treatments for no measurable benefit.

Most men with elevated levels of PSA have benign prostate conditions and do not have cancer. Workers in the field have developed blood, urine and tissue tests to try to address the need for more specific detection of PCa over benign diseases including Benign Prostatic Hyperplasia (BPH), Prostatic Intraepithelial Neoplasia (PIN) and Atypical Small Acinar Proliferation (ASAP). These tests include serial PSA measurement, the Prostate Health Index, the 4K score and Prostate cancer antigen 3. The Prostate Health Index and the 4K score also provide some information on the differentiation between high and low grade PCa.

Serial PSA measurement is based on the finding that PCa is associated with rising PSA levels as well as high absolute levels.

The Prostate Health Index (Phi) is calculated as Phi = (p2PSA/fPSA x √PSA) where p2PSA is proprostate-specific antigen and fPSA is free PSA. In studies the Phi increased the AUC (Area Under the Curve) for ROC curves (Receiver Operator Curves) for detection of PCA from 56% for PSA to 74% for Phi. Furthermore, if a high cut-off was used, approximately 50% of the Phi positive men were found to have PCA of Gleason score ≥7. However, the sensitivity for high grade PCa at this cut-off was 61% meaning 39% of high grade cancers would be missed (Stephan et al; 2013).

The 4K score measures 4 kallikrein moieties in the blood (Total PSA, Free PSA, Intact PSA and human glandular kallikrein 2) and combines these results with the patient's clinical information and the results of previous biopsies in an algorithm to predict the risk of having high-grade PCa versus indolent cancer or no cancer.

The Prostate cancer antigen 3 (PCA3) test measures a noncoding messenger RNA that has been shown to be elevated in the urine of men with PCa, but not in men with normal prostatic glands or in BPH. PCA3 has higher AUC values (66-72%) than PSA (54-63%) for PCa detection and was approved by the FDA as a test for PCa in the context of a previous negative prostate biopsy. PCA3 is also considered helpful in deciding when to re-biopsy and in the follow-up of patients under active surveillance (Crawford et al; 2014).

Workers have also developed genomic tissue tests to be performed on biopsy material to better stratify patient risk (Cuchiarra et al; 2018). ConfirmMDx is a three gene epigenetic methylation assay which is performed on biopsy tissue material from negative biopsies to confirm they are truly negative with a high negative predictive value of >90% to help reduce the need for repeat biopsies (Crawford et al; 2014). Similarly, the Oncotype DX Prostate Cancer Assay measures the expression of 12 cancer-related genes and five reference genes in biopsy tissue and combines these in an algorithm to calculate the Genomic Prostate Score. Together with PSA level, Gleason score and TNM stage, the Genomic Prostate Score improves risk stratification of PCa into very low, low and modified intermediate risk to help clinicians select appropriate candidates for active surveillance.

Workers in the field have also investigated numerous other biomarkers for the detection of and/or stratification of PCa including circulating cell free nucleosomes (as referenced in WO2005019826, WO2013030577, WO2013030579 and WO2013084002). Scanning methods have also been developed including multi-parametric MRI scans to identify men who do not have PCa to avoid unnecessary prostate biopsies.

The role of IL-6 in tumorigenesis has been well-established in a wide range of human cancers including PCa, Colorectal Cancer (CRC), lymphoma, glioma, melanoma, breast, ovarian, renal and pancreatic cancer (Wang and Sun; 2014). Elevated circulating IL-6 levels are associated with tumorigenesis, disease progression and poor prognosis in many cancers including PCa, CRC, skin, breast, lung, oesophageal, liver, pancreatic, gastric, gynaecological, kidney, bladder and haematological cancers (Taniguchi and Karin; 2014).

IL-8 is also known to be associated with many cancers including PCa (Xie; 2001). Circulating IL-8 levels were shown to be increased in many cancers including PCa, CRC, gastric, melanoma, ovarian, pancreatic and breast cancer. Circulating IL-8 levels were reported to rise from 6.8 pg/ml in normal men to 15.6 pg/ml in PCa patients with clinical Stages A to C and to 27.8 pg/ml patients with metastatic PCa (Veltri et al; 1999). More recently Chadha et al; 2014, reported that median circulating IL-8 levels rose significantly (P < 0.001) in PCa from 4.00 pg/ml in healthy men to 8.13 pg/ml in men with elevated PSA levels but a negative prostate biopsy to 16.90 pg/ml in men with localized PCa and to 43.50 pg/ml in men with castration resistant PCa. Moreover, increased cellular IL-8 production by PCa cells has been shown to have a role in malignant PCa disease progression. Increased intracellular IL-8 levels led to increasing tumorigenicity of PCa cells when injected into mice and IL-8 depletion led to lower tumorigenicity (Xu et al; 2009). Similarly, high IL-8 production by PCa cells was shown to promote tumour cell survival and resistance to cytotoxic drugs and radiotherapy. In contrast, low intracellular IL-8 levels were reported to lead to lowered resistance to radiotherapy (Xu et al; 2012), lowered resistance to cytotoxic chemotherapeutic drugs including Docetaxel, Staurosporine and Rapamycin and cause cell cycle arrest and apoptosis (Singh and Lokeshwar; 2009).

Findings are similar in other cancers. A meta-analysis of 18 articles on the accuracy of IL-8 levels for CRC diagnosis reported a consensus that elevated IL-8 serum levels have a high diagnostic accuracy for CRC with a sensitivity of 0.70 and specificity 0.91 and are also significantly correlated with poor CRC prognosis (Xia et al; 2015). Moreover, as with PCa cells, high IL-8 levels in CRC cells were shown to be associated with cytotoxic drug resistance (Du et al; 2018).

Disturbances in the balance of circulating interleukin levels have been reported for subjects diagnosed with gastric cancer including both the diffuse and intestinal gastric cancer types, as defined by the Lauren classification, as well as mixed type gastric cancer (Madej-Michniewicz et al; 2015). In gastric cancer, circulating IL-6 levels were observed to rise, but circulating IL-8 and interleukin-10 (IL-10) levels were lower than in healthy subjects. However, this effect was not shown to occur for other gastrointestinal cancer types investigated by the same author, including pancreatic cancer, lymphomas, gastrointestinal stromal tumours and neuroendocrine neoplasms.

Batra et al. (2014) is a review article describing the quest to identify prostate cancer circulating biomarkers with bench-to-bedside potential. Komatsu et al. (2011) describes gene expression profiles in peripheral blood as a biomarker in cancer patients receiving peptide vaccination. WO9949083 discloses diagnostic techniques for the detection of human disease states that affect gene expression in peripheral leukocytes.

Despite the advances described, PSA remains the main non-invasive test used in the detection, stratification and management of PCa. Overdiagnosis of prostate cancer is a major difficulty in the management of prostate disease. The PSA test is moderately effectively for the detection of PCa in men. However, it is a poor indicator of which men need treatment. There is a need for non-invasive methods for patient stratification to identify men with high grade PCa who are in need of treatment. An ideal test would correlate with Gleason scores and/or to the clinical risk stratification including Gleason score, TNM stage and PSA. Such a method has the potential to improve the diagnosis and management of prostate cancer patients, to reduce unnecessary invasive biopsies and overtreatment in men with low grade disease and to reduce costs to healthcare providers. It would also be useful for non-invasive monitoring of patients with low grade disease for watchful waiting and/or active surveillance, deciding when to re-biopsy and for the detection of patient relapse post-treatment or post-prostatectomy.

### SUMMARY OF THE INVENTION

According to a first aspect of the invention, there is provided the use of Interleukin-8 as a biomarker in a blood, serum or plasma sample for diagnosing and/or monitoring prostate cancer, wherein a lower level of Interleukin-8 compared to a control is indicative of the presence and/or progression of prostate cancer.

According to a further aspect of the invention, there is provided a method of diagnosing prostate cancer in a patient, comprising:
detecting or measuring the level of Interleukin-8, optionally in combination with at least one biomarker selected from the list consisting of: PSA and nucleosomes or a component thereof, in a blood, serum or plasma sample obtained from the patient,
wherein a lower level of Interleukin-8 compared to a control is indicative of the presence of prostate cancer.

According to a further aspect of the invention, there is provided a method for assessing the suitability of a patient for a prostate biopsy, comprising:
detecting or measuring the level of Interleukin-8, optionally in combination with at least one biomarker selected from the list consisting of: PSA and nucleosomes or a component thereof, in a blood, serum or plasma sample obtained from the patient; and
wherein a lower level of Interleukin-8 compared to a control is indicative that the patient requires a prostate biopsy.

According to a further aspect of the invention, there is provided the use of a panel comprising reagents to detect Interleukin-8, Interleukin-6 and nucleosomes or a component thereof, according to the use or methods as defined herein.

### BRIEF DESCRIPTION OF THE FIGURES

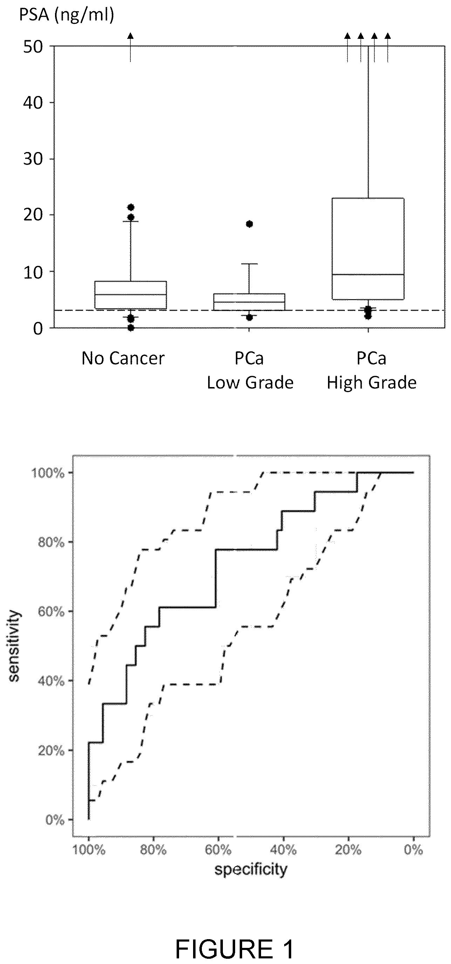

**Figure 1****:** Variation in PSA levels (ng/ml) measured in men referred for prostate biopsy who were subsequently found to have no cancer, low grade or high grade cancer on biopsy. The horizontal dashed line indicates a PSA level of 3 ng/ml. Vertical arrows indicate samples with high or low levels off the scale of the graph. The ROC curve shows the discrimination of PSA for men subsequently diagnosed with high grade PCa from other men referred for biopsy with low grade cancer or no cancer. The dashed lines on the ROC curve represent the 95% error limits on the curve.

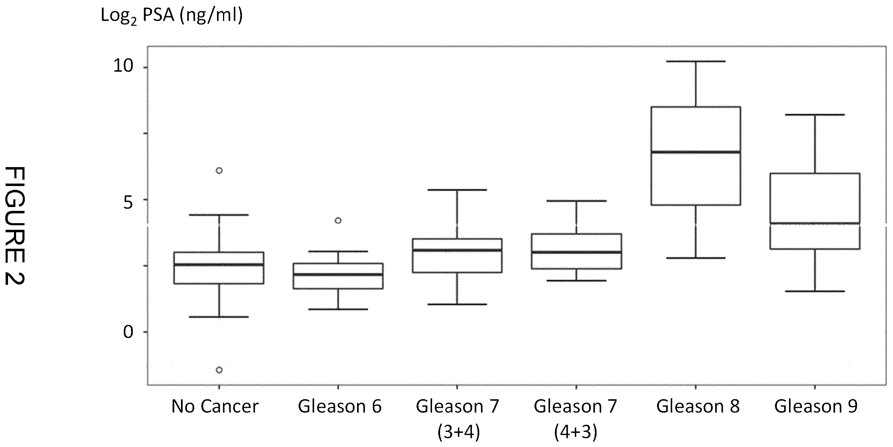

**Figure 2****:** Variation in PSA levels (ng/ml) with Gleason grade determined on prostate biopsy.

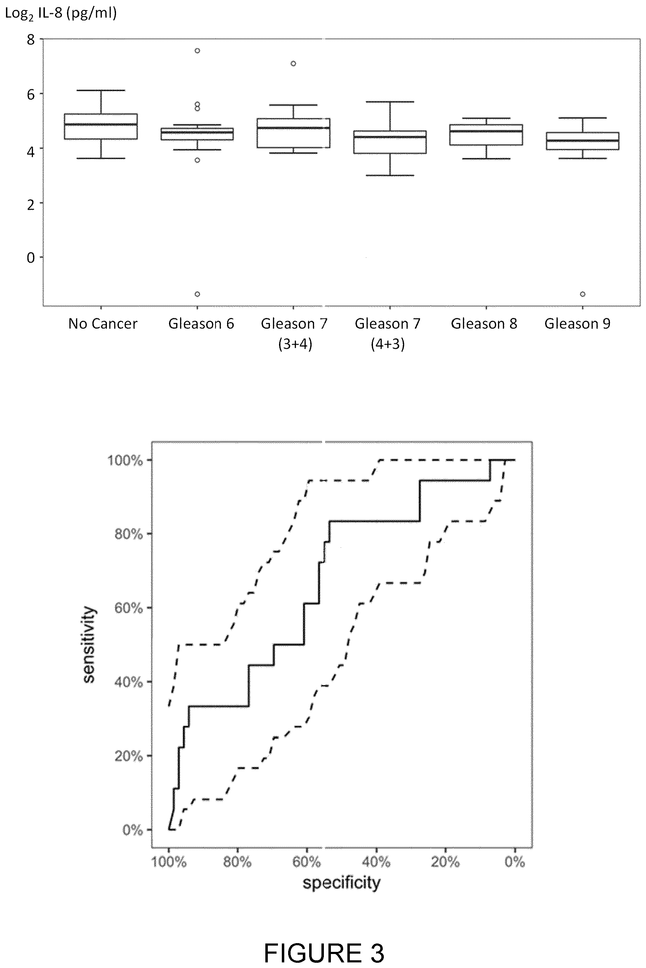

**Figure 3****:** Variation in IL-8 levels (pg/ml) with Gleason grade determined on prostate biopsy. The ROC curve shows the discrimination of IL-8 for men subsequently diagnosed with high grade PCa from other men referred for biopsy with low grade cancer or no cancer.

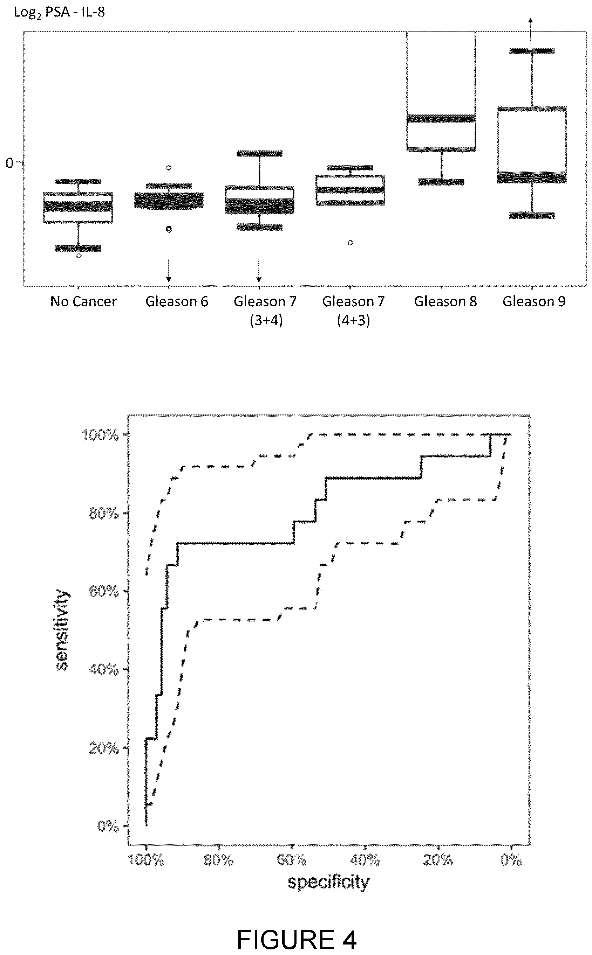

**Figure 4****:** Variation in the difference between PSA levels (ng/ml) and IL-8 levels (pg/ml) with Gleason grade determined on prostate biopsy. The ROC curve shows the discrimination of [PSA - IL-8] for men subsequently diagnosed with high grade PCa from other men referred for biopsy with low grade cancer or no cancer.

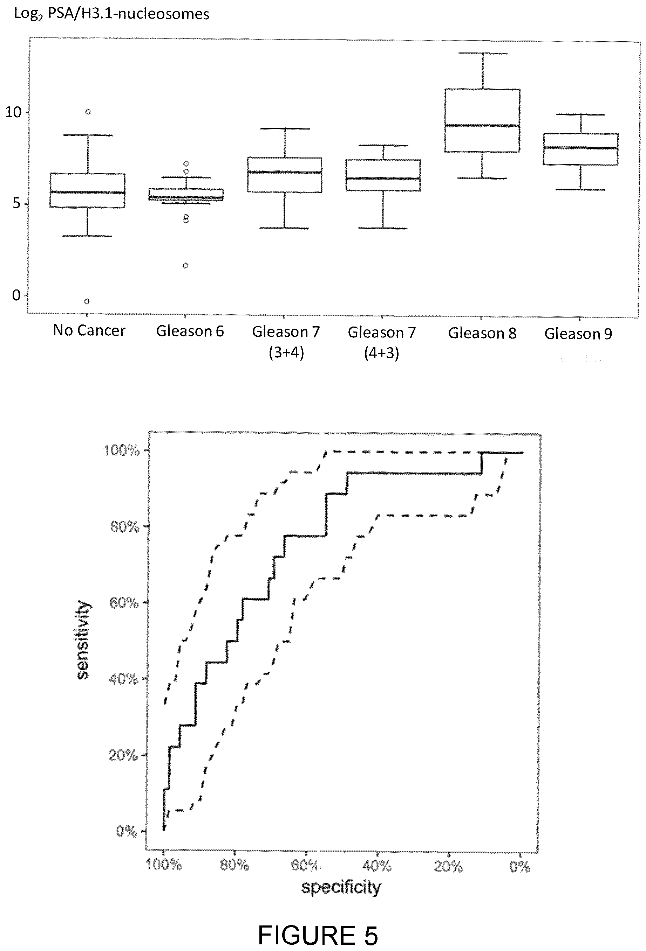

**Figure 5****:** Variation in the results of a ratio of [PSA/H3.1-nucleosome level] with Gleason grade determined on prostate biopsy. The ROC curve shows the discrimination of the ratio for men subsequently diagnosed with high grade PCa from other men referred for biopsy with low grade cancer or no cancer.

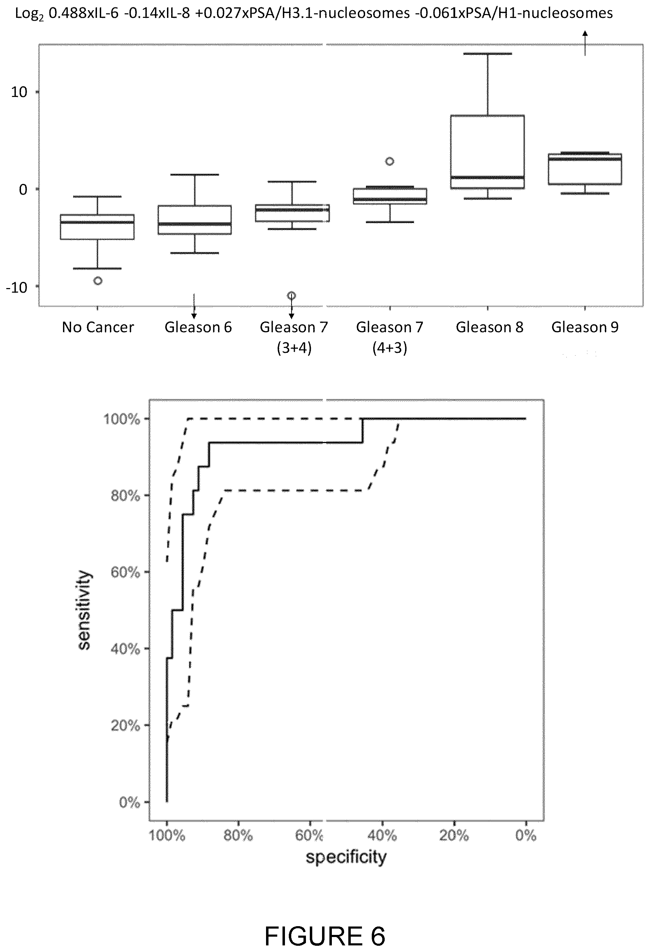

**Figure 6****:** Results of an IL-8, IL-6, PSA, H3.1-nucleosome and H1-nucleosome panel (using the algorithm "Panel Score = 0.49[IL-6] -0.14[IL-8] +0.061[PSA/H3.1-nucleosomes] - 0.027[PSA/H1-nucleosomes]") with Gleason grade determined on prostate biopsy. The ROC curve shows the discrimination of the algorithm for men subsequently diagnosed with high grade PCa from other men referred for biopsy with low grade cancer or no cancer.

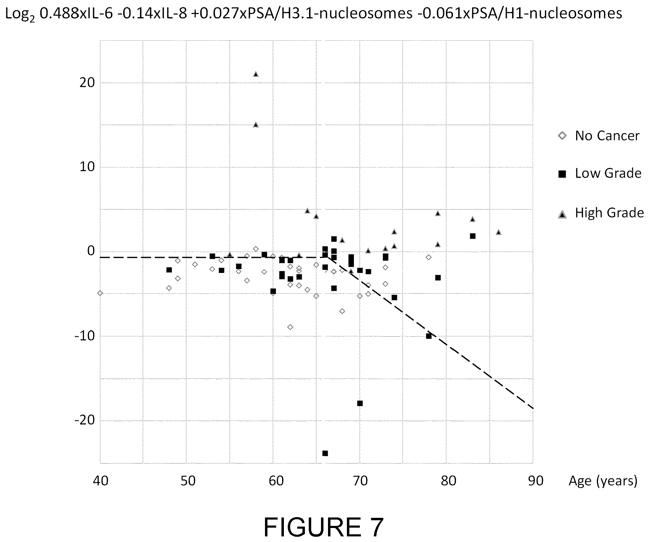

**Figure 7****:** Results of an IL-8, IL-6, PSA, H3.1-nucleosome and H1-nucleosome panel (using the algorithm "Panel Score = 0.49[IL-6] -0.14[IL-8] +0.027[PSA/H3.1-nucleosomes] - 0.061[PSA/H1-nucleosomes]") plotted against patient age. The line drawn represents a continuous age dependent cut-off that may be used as the basis of a rule-out test to identify men that do not have high grade PCa (no patient below the line was found to have high grade PCa).

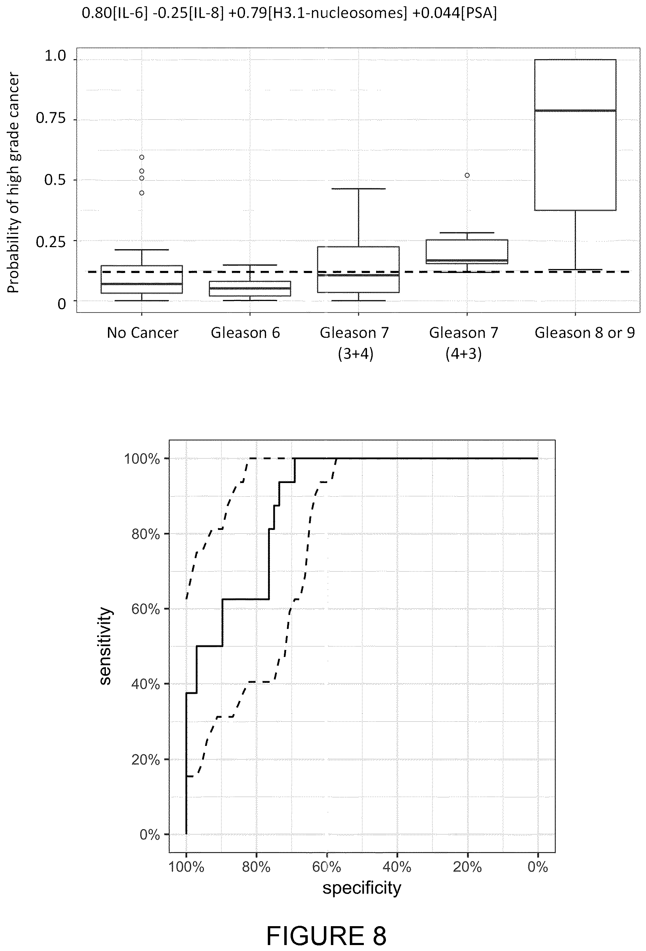

**Figure 8****:** Results of a regression analysis algorithm of the form "Panel Score = 0.80[IL-6] -0.25[IL-8] +0.79[H3.1-nucleosomes] +0.044[PSA]" expressed as the probability of each patient having a high grade cancer plotted against Gleason grade determined on prostate biopsy. The dashed line shows the algorithm cut-off threshold below which no patient was observed to have high grade cancer with Gleason score ≥7(4+3). The ROC curve shows the discrimination of the algorithm for men subsequently diagnosed with high grade PCa from other men referred for biopsy with low grade cancer or no cancer (AUC=89%). All nucleosome and interleukin level measurements were made in plasma. PSA level was measured in serum.

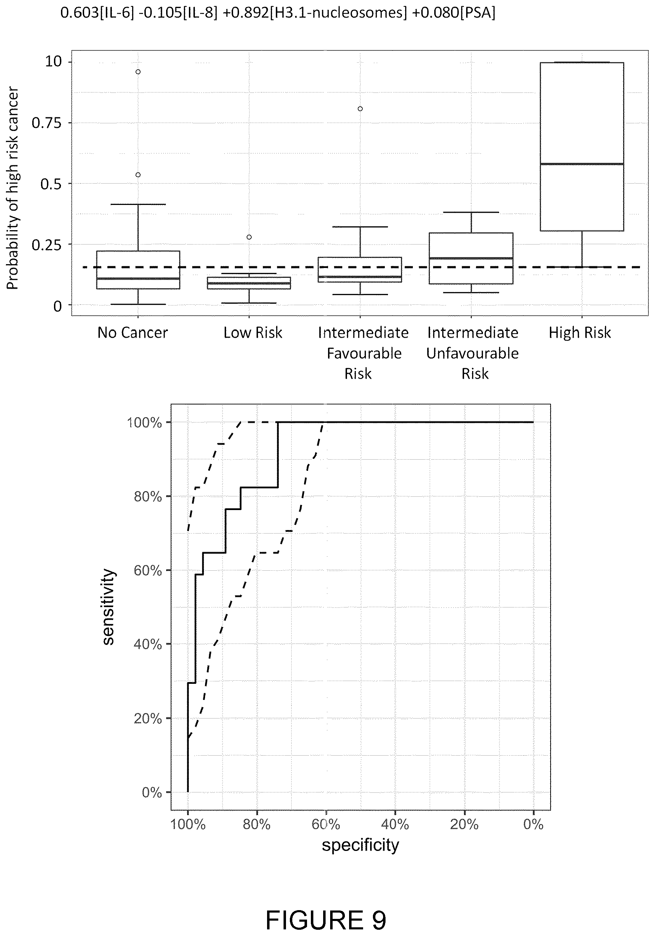

**Figure 9****:** Results of a regression analysis algorithm for plasma measurements of the form "Panel Score = 0.603[IL-6] -0.105[IL-8] +0.892[H3.1-nucleosomes] +0.080[PSA]" expressed as the probability of each patient having a High Risk cancer plotted against the risk level determined on prostate biopsy. The dashed line shows the algorithm cut-off threshold below which no patient was observed to have high risk cancer. The ROC curve shows the discrimination of the algorithm for men subsequently diagnosed with High Risk PCa from other men referred for biopsy with Low Risk PCa or no cancer (AUC=92%). All nucleosome and interleukin level measurements were made in plasma. PSA level was measured in serum.

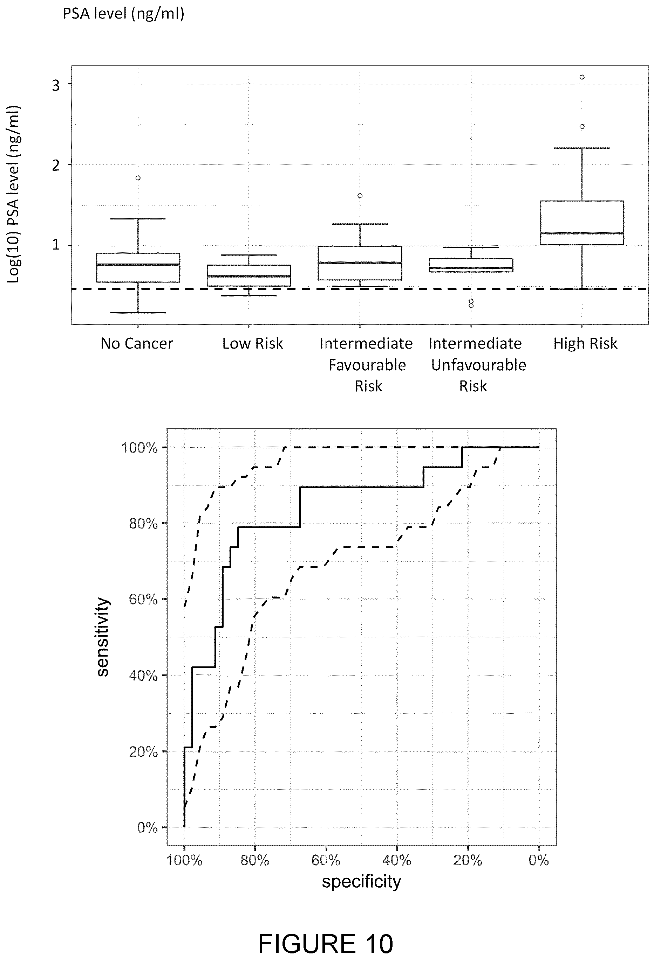

**Figure 10****:** PSA results expressed in Log(10) form plotted against the risk level determined on prostate biopsy. The dashed line shows the PSA cut-off threshold below which no patient was observed to have high risk cancer. The ROC curve shows the discrimination of PSA level for men subsequently diagnosed with High Risk PCa from other men referred for biopsy with Low Risk PCa or no cancer (AUC=84%). PSA level was measured in serum.

### DETAILED DESCRIPTION OF THE INVENTION

Surprisingly, we have shown that, whilst there is an increase in IL-6 levels, there is a decrease in circulating IL-8 levels in patients with high grade or high risk prostate cancer (PCa) compared to men with low risk PCa or men with no PCa, and these two inflammatory molecules can be used to detect PCa. We now report the development of non-invasive blood tests and methods of treatment based on PSA, nucleosome and inflammatory marker measurements that accurately differentiate men with high grade PCa from those with low grade PCa or no cancer among men referred for prostate biopsy. Moreover, the test results numerically correlate with patient Gleason scores. Similarly, the marker measurements accurately differentiate men with high risk PCa from those with low risk PCa or no cancer among men referred for prostate biopsy, and the test results numerically correlate with patient risk categories determined on biopsy.

We performed a PCa clinical study for circulating IL-6, IL-8 and nucleosome measurements. The study involved 84 randomly selected men referred for prostate biopsy to investigate suspected PCa. Biopsy findings were negative for 35 men with no cancer. Biopsy findings were positive in 49 men of whom 16 men had PCa with Gleason grade 6, 17 men with grade 7(3+4), 6 men with grade 7(4+3), 3 men with grade 8, and 7 men with grade 9. We measured serum levels of PSA, nucleosomes containing histone variant H3.1 (H3.1-nucleosomes), nucleosomes containing histone H1 (H1-nucleosomes), IL-6 and IL-8 in blood samples taken from the men. The vast majority of the men referred for biopsy had elevated PSA levels including those subsequently found to have no cancer on biopsy **(****Figures 1** and **2****).**

We also show that the use of a combined PSA measurement with an interleukin level measurement and/or a nucleosome level measurement in blood, serum or plasma may be used as a biomarker panel for prostate cancer (PCa) wherein the combined measurement may take the form of a difference in circulating PSA and interleukin levels, a ratio of circulating PSA/Interleukin levels or a ratio of circulating PSA/nucleosome levels that is useful for the identification of patients with aggressive/high grade PCa. These men are in need of biopsy and treatment by surgery, radiotherapy, chemotherapy or other methods. Men with low grade disease can be monitored and men with no disease may not require further treatment.

Therefore, according to a first aspect of the invention, there is provided the use of Interleukin-8 as a biomarker in a blood, serum or plasma sample for diagnosing and/or monitoring prostate cancer, wherein a lower level of Interleukin-8 compared to a control is indicative of the presence and/or progression of prostate cancer.

Interleukin-8 (IL-8, also known as CXCL8) is a chemokine that attracts immune cells, such as neutrophils, basophils, and T-cells. It is released from several cell types in response to an inflammatory stimulus. The sequence of human IL-8 is known in the art and is described at UniProt Accession No. P10145.

It will be clear to those skilled in the art that the control subjects may be selected on a variety of basis which may include, for example, subjects known to be free of the disease or may be subjects with a different disease (for example, for the investigation of differential diagnosis). The "control" may comprise a healthy subject, a non-diseased subject and/or a subject without cancer. The present invention is particularly suited for detecting patients with high grade prostate cancer, therefore in this embodiment the control may also be a subject with low grade prostate cancer. Comparison with a control is well known in the field of diagnostics.

It will be understood that it is not necessary to measure healthy/non-diseased controls for comparative purposes on every occasion because once the 'normal range' is established it can be used as a benchmark for all subsequent tests. A normal range can be established by obtaining samples from multiple control subjects without cancer and testing for the level of biomarker. Results *(i.e.* biomarker levels) for subjects suspected to have cancer can then be examined to see if they fall within, or outside of, the respective normal range. Use of a 'normal range' is standard practice for the detection of disease.

If a subject is determined to not have prostate cancer, then the invention may still be used for the purposes of monitoring disease progression. For example, if the use comprises a blood, serum or plasma sample from a subject determined not to have prostate cancer or to have low grade prostate cancer, then the biomarker level measurement can be repeated at another time point to establish if the biomarker level has changed.

In one embodiment, the use additionally comprises Interleukin-6 as a biomarker, wherein a higher level of Interleukin-6 compared to a control is indicative of the presence and/or progression of prostate cancer.

Interleukin-6 (IL-6) is a cytokine with a wide variety of biological functions. It is a potent inducer of fever and the acute phase response. The sequence of human IL-6 is known in the art and is described at UniProt Accession No. P05231.

In one embodiment, the use additionally comprises one or more biomarkers selected from the list consisting of: PSA and nucleosomes or a component thereof. In a further embodiment, the component of a nucleosome is an epigenetic feature of a nucleosome. In a yet further embodiment, the epigenetic feature of a nucleosome is selected from: a post-translational modification, histone variant/isoform, modified nucleotide and/or a protein bound to a nucleosome *(i.e.* a nucleosome-protein adduct), in particular a histone variant/isoform. In a yet further embodiment, the histone variant/isoform is a variant/isoform of histone H3, such as histone H3.1.

PSA (Prostate Specific Antigen, also known as gamma-seminoprotein or kallikrein-3) is a biomarker currently used in the art where elevated levels in the serum have been associated with the presence of prostate cancer or other prostate disorders. A high blood level of PSA (typically greater than 3 ng/ml) indicates that the patient should be referred for biopsy. In one embodiment, the use additionally comprises PSA as a biomarker.

In one embodiment, the use additionally comprises nucleosomes or a component thereof as a biomarker. Said nucleosomes may be detected as circulating nucleosomes in the blood, serum or plasma sample, *i.e.* they are cell free nucleosomes.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

References to "nucleosomes *per* se" refers to the total nucleosome level present in the sample. The total nucleosome level can be detected by detecting a histone protein common to all nucleosomes, such as histone H4. As previously reported in WO2016067029, nucleosomes containing particular histone variants/isoforms, such as histone H3.1, H3.2 or H3t, may be used to isolate cell free nucleosomes originating from tumour cells. Therefore, epigenetic subsets of cell free nucleosomes including those of tumour origin may be detected. The term "component thereof" as used herein refers to a part of the nucleosome, *i.e.* the whole nucleosome does not need to be detected. For example, in one embodiment the component thereof is histone H1. In one embodiment, the component of the nucleosome is an epigenetic feature (of the nucleosome). Thus, in one embodiment, the use additionally comprises nucleosomes *per se,* nucleosomes containing histone H3.1 and/or histone H1 as biomarkers.

References to "nucleosome" may refer to "cell free nucleosome" when detected in body fluid samples. It will be appreciated that the term "cell free nucleosome" used throughout this document is intended to include any cell free chromatin fragment that includes one or more nucleosomes. Epigenetic signal structures/features of a cell free nucleosome as referred herein may comprise, without limitation, one or more histone post-translational modifications, histone isoforms, modified nucleotides and/or proteins bound to a nucleosome in a nucleosome-protein adduct.

Normal cell turnover in adult humans involves the creation by cell division of some 10¹¹ cells daily and the death of a similar number, mainly by apoptosis. During the process of apoptosis chromatin is broken down into mononucleosomes and oligonucleosomes which are released from the cells. Under normal conditions the levels of circulating nucleosomes found in healthy subjects is reported to be low. Elevated levels are found in subjects with a variety of conditions including many cancers, auto-immune diseases, inflammatory conditions, stroke and myocardial infarction (Holdenrieder & Stieber, 2009).

Mononucleosomes and oligonucleosomes can be detected by Enzyme-Linked ImmunoSorbant Assay (ELISA) and several methods have been reported (Salgame et al. (1997); Holdenrieder et al. (2001); van Nieuwenhuijze et al. (2003); WO2005019826; WO2013030577; WO2013030579; and WO2013084002). These assays typically employ an anti-histone antibody (for example anti-H2B, anti-H3 or anti-H1, H2A, H2B, H3 and H4) as capture antibody and a detection antibody (which varies depending upon the moiety to be detected). In one embodiment, the anti-histone antibody comprises an anti-H3 antibody or an anti-H1 antibody.

Circulating nucleosomes are not a homogeneous group of protein-nucleic acid complexes. Rather, they are a heterogeneous group of chromatin fragments originating from the digestion of chromatin on cell death and include an immense variety of epigenetic structures including particular histone isoforms or variants, post-translational histone modifications, nucleotides or modified nucleotides and nucleosome adducts. It will be clear to those skilled in the art that an elevation in nucleosome levels will be associated with elevations in some circulating nucleosome subsets containing particular epigenetic signals including nucleosomes comprising particular histone isoforms or variants, comprising particular post-translational histone modifications, comprising particular nucleotides or modified nucleotides and comprising particular nucleosome adducts. Assays for these types of chromatin fragments are known in the art (for example, see WO2005/019826, WO2013/030579, WO2013/030578, WO2013/084002).

In one embodiment, the epigenetic feature of the nucleosome is selected from the group consisting of: a post-translational histone modification, a histone variant, a particular nucleotide and a protein adduct. It will be understood that the terms "epigenetic signal structure" and "epigenetic feature" are used interchangeably herein. They refer to particular features of the nucleosome that may be detected.

In one embodiment, the epigenetic feature of the nucleosome comprises one or more histone post-translational modifications. The structure of nucleosomes can vary by Post Translational Modification (PTM) of histone proteins. PTM of histone proteins typically occurs on the tails of the core histones and common modifications include acetylation, methylation or ubiquitination of lysine residues as well as methylation of arginine residues and phosphorylation of serine residues and many others. Histone modifications are known to be involved in epigenetic regulation of gene expression (Herranz and Esteller (2007)). For example, in one embodiment, the post-translational histone modification is H3K9Me3.

In one embodiment of the invention a group or class of related histone modifications (rather than a single modification) is detected. A typical example of this embodiment, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (e.g. a Pan-acetyl H4 antibody), anti-citrullination antibodies or anti-ubiquitination antibodies.

In one embodiment, the epigenetic feature of the nucleosome comprises one or more histone variants or isoforms. The term "histone variant" and "histone isoform" may be used interchangeably herein. The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone Database (Mariño-Ramírez, L., Levine, K.M., Morales, M., Zhang, S., Moreland, R.T., Baxevanis, A.D., and Landsman, D. The Histone Database: an integrated resource for histones and histone fold-containing proteins. Database Vol.2011. and http://genome.nhgri.nih.gov/histones/complete.shtml), the GenBank (NIH genetic sequence) Database, the EMBL Nucleotide Sequence Database and the DNA Data Bank of Japan (DDBJ). For example, variants of histone H2 include H2A1, H2A2, mH2A1, mH2A2, H2AX and H2AZ. As a further example, variants of histone H3 include H3.1, H3.2 and H3t.

In one embodiment, the epigenetic feature of the nucleosome comprises one or more DNA modifications. In addition to the epigenetic signalling mediated by nucleosome histone isoform and PTM composition, nucleosomes also differ in their nucleotide and modified nucleotide composition. Global DNA hypomethylation is a hallmark of cancer cells and some nucleosomes may comprise more 5-methylcytosine residues (or 5-hydroxymethylcytosine residues or other nucleotides or modified nucleotides) than other nucleosomes. In one embodiment, the DNA modification is selected from 5-methylcytosine or 5-hydroxymethylcytosine.

In one embodiment, the epigenetic feature of the nucleosome comprises one or more protein-nucleosome adducts or complexes. A further type of circulating nucleosome subset is nucleosome protein adducts. It has been known for many years that chromatin comprises a large number of non-histone proteins bound to its constituent DNA and/or histones. These chromatin associated proteins are of a wide variety of types and have a variety of functions including transcription factors, transcription enhancement factors, transcription repression factors, histone modifying enzymes, DNA damage repair proteins and many more. These chromatin fragments including nucleosomes and other non-histone chromatin proteins or DNA and other non-histone chromatin proteins are described in the art.

In one embodiment, the protein adducted to the nucleosome (and which therefore may be used as a biomarker) is selected from: a transcription factor, a High Mobility Group Protein or chromatin modifying enzyme. References to "transcription factor" refer to proteins that bind to DNA and regulate gene expression by promoting (i.e. activators) or suppressing (i.e. repressors) transcription. Transcription factors contain one or more DNA-binding domains (DBDs), which attach to specific sequences of DNA adjacent to the genes that they regulate. All of the circulating nucleosomes and nucleosome moieties, types or subgroups described herein may be useful in the present invention.

It will be understood that methods and uses of the present invention find particular use in blood, serum or plasma samples obtained from a patient. In one embodiment, the sample is a blood, blood plasma or blood serum sample. In a further embodiment, the sample is a plasma sample.

In one embodiment, the biomarkers are for use in diagnosing the grade of prostate cancer. Prostate cancer can be separated into low grade or high grade. Men with low grade prostate cancer are generally not treated for cancer but are monitored to check that the cancer remains low risk. High grade cancers are considered high risk, aggressive and potentially lethal, therefore men with high grade cancers are generally treated. In a further embodiment, the stage of prostate cancer is high grade prostate cancer.

It has been found that the biomarker panels described herein correlate well with the Gleason grading system. In the Gleason grading system where the cancer tissue is graded on a scale from 1-5. A cancer tissue that is similar in appearance to normal prostate tissue under the microscope may be assigned a grade of 1. Cancer tissues that deviate from the features of normal cells will be graded higher and those that deviate greatly from normal cells will be graded 5. Two Gleason grades are assigned to (i) the most predominant cancer tissue in the biopsy material and (ii) the highest grade other tissue. These two numbers are added together to give a Gleason score ranging from 2-10. For example, a Gleason score of 7 may be derived as (3+4), where most of the cancer seen is grade 3 and the highest grade of any other cancer seen is grade 4, or (4+3). Cancers with a Gleason score of 7 (3+4) or below are considered low grade cancers. Cancers with a Gleason score of 7 (4+3) or above are considered high grade cancers.

In one embodiment, the biomarkers are for use in risk stratification of a patient with suspected prostate cancer. In a further embodiment, the patient has high risk prostate cancer. The biomarkers may be for use in predicting or diagnosing the risk classification of a prostate cancer, *i.e.* predicting or diagnosing a patient with low risk, intermediate risk or high risk prostate cancer. Prostate cancer can be separated into various categories of increasing risk from low risk to high risk. Examples of risk stratification systems include those of the Memorial Sloan-Kettering and Seattle groups (Humphrey; 2014 and Barry and Newman; 2015) and the National Comprehensive Cancer Network (NCCN) Clinical Practice Guidelines in Oncology for Prostate Cancer (NCCN; 2018). Men with low risk prostate cancer are generally not treated for cancer but are monitored to check that the cancer remains low risk. High risk cancers are considered high risk, aggressive and potentially lethal, therefore men with high risk cancers are generally treated. Men with intermediate risk prostate cancer (which can be further separated into intermediate favourable risk or intermediate unfavourable risk) may be treated if appropriate depending on other factors and the input of the patient.

It has been found that the biomarker panels described herein produce results and median results that correlate well with the clinical risk categories as shown in Figure 9. The median biomarker result increases from low risk, to intermediate favourable risk, to intermediate unfavourable risk to high risk PCa. The biomarker panel in Figure 9 was able to distinguish most men (65%) with high risk disease at high specificity (95%) and was also able to rule out high risk disease in most men with no cancer or low risk disease (>70%) with no false negative cases *(i.e.* without missing any high risk cancer cases). This discrimination was higher than that observed for the PSA test (22%) shown in Figure 10. Thus, biomarker tests of the invention predict the clinical risk category of most patients and can be used to help stratify patients according to risk, and may be used to avoid false positives and overdiagnosis in PCa and therefore to avoid unnecessary biopsies in many men. The tests of the invention may also be used serially to monitor men with low risk PCa for disease progression.

In one embodiment, the biomarkers (or methods) described herein are for use in identifying patients with high risk prostate cancer, for example patients with a Gleason score of 7 (4+3) or above.

According to a further aspect of the invention, there is provided a method of diagnosing prostate cancer in a patient, comprising:
detecting or measuring the level of Interleukin-8, optionally in combination with at least one biomarker selected from the list consisting of: PSA and nucleosomes or a component thereof, in a blood, serum or plasma sample obtained from the patient,
wherein a lower level of Interleukin-8 compared to a control is indicative of the presence of prostate cancer.

In one embodiment, the method comprises detecting or measuring the level of Interleukin-8 and Interleukin-6, optionally in combination with at least one biomarker selected from the list consisting of: PSA and nucleosomes or a component thereof, in a blood, serum or plasma sample obtained from the patient, wherein a lower level of Interleukin-8 and a higher level of Interleukin-6 compared to a control is indicative of the presence of prostate cancer.

The invention finds particular use in assessing whether a patient requires a prostate biopsy. Prostate biopsies are invasive and are relatively costly to healthcare providers, therefore there is a need to reduce the number of patients sent for unnecessary biopsies. Therefore, according to a further aspect of the invention, there is provided a method for assessing the suitability of a patient for a prostate biopsy, comprising:
detecting or measuring the level of Interleukin-8, optionally in combination with at least one biomarker selected from the list consisting of: PSA and nucleosomes or a component thereof, in a blood, serum or plasma sample obtained from the patient; and
wherein a lower level of Interleukin-8 compared to a control is indicative that the patient requires a prostate biopsy.

In one embodiment, the method comprises detecting or measuring the level of Interleukin-8 and Interleukin-6, optionally in combination with at least one biomarker selected from the list consisting of: PSA and nucleosomes or a component thereof, in a blood, serum or plasma sample obtained from the patient; wherein a lower level of Interleukin-8 and a higher level of Interleukin-6 compared to a control is indicative that the patient requires a prostate biopsy.

According to a further aspect of the invention, there is provided a method of risk stratification of a patient with suspected prostate cancer, comprising:
detecting or measuring the level of Interleukin-8, optionally in combination with at least one biomarker selected from the list consisting of: PSA and nucleosomes or a component thereof, in a blood, serum or plasma sample obtained from the patient; and
categorising the patient as high risk, intermediate risk or low risk,
wherein a lower level of Interleukin-8 compared to a control is used to categorise a patient as high risk.

In one embodiment, the method comprises detecting or measuring the level of Interleukin-8 and Interleukin-6, optionally in combination with at least one biomarker selected from the list consisting of: PSA and nucleosomes or a component thereof, in a blood, serum or plasma sample obtained from the patient; and categorising the patient as high risk, intermediate risk or low risk; wherein a lower level of Interleukin-8 and a higher level of Interleukin-6 compared to a control is used to categorise a patient as high risk.

The method of the invention has uses in avoiding unnecessary biopsies and may also be used to improve disease outcomes by improved risk stratification of prostate cancer patients by inclusion of the methods in the risk stratification process. It will be understood that if a patient is categorised as high risk, then they should be subsequently treated, whereas a patient categorised as low risk does not need to be treated, but can be actively monitored.

In one embodiment, the method additionally comprises determining at least one clinical parameter for the patient. This parameter can be used in the interpretation of results. Clinical parameters may include any relevant clinical information for example, without limitation, gender, weight, Body Mass Index (BMI), smoking status and dietary habits. Therefore, in one embodiment, the clinical parameter is selected from the group consisting of: age, sex and body mass index (BMI).

Age was found to be an independent predictor of high grade PCa and identified 39% of men with high grade PCa from all others at 90% specificity with AUC=72%. Less than 20% of men with no cancer or low grade cancer could be identified by age alone as not in need of a prostate biopsy without missing any high grade cancers. Age was found to contribute usefully as a parameter in many algorithms or models tested. Some models/algorithms derived using results obtained in serum samples, together with their AUC, for the discrimination of men with high Gleason grade PCa from all others (men with low Gleason grade PCa or no cancer) are shown in Table 1.

**Table 1. Example test panels and algorithms with observed AUC and p-values**

| Panel and Algorithm | AUC | p |
|---|---|---|
| Score = a[IL-6] -b[IL-8] +c[PSA/H3.1-nucleosomes] +d[PSA/H1-nucleosomes] | 94% | <0.01 |
| Score = -a[IL-8] +b[PSA/H3.1-nucleosomes] +c[PSA/H1-nucleosomes] | 89% | <0.05 |
| Score = a[IL-6] +b[PSA/H3.1-nucleosomes] +c[PSA/H1-nucleosomes] | 87% | <0.01 |
| Score = a[age] +b[PSA/H3.1-nucleosomes] +c[PSA/H1-nucleosomes] | 87% | <0.05 |
| Score = a[age] -b[IL-8] +c[PSA/H3.1-nucleosomes] | 85% | <0.02 |
| Score = -a[IL-8] +b[PSA/H1-nucleosomes] | 81% | <0.03 |
| Score = a[PSA] -b[IL-8] | 81% | <0.04 |
| Score = a[PSA/IL-8] | 79% | <0.02 |
| Score = a[PSA/H3.1-nucleosomes] +B[PSA/H1-nucleosomes] | 79% | <0.03 |
| Score = a[IL-8] +b[PSA/H3.1-nucleosomes] | 79% | <0.02 |
| Score = a[age] -b[IL-8] | 79% | <0.03 |
| Score = a[PSA/H3.1-nucleosomes] | 77% | <0.02 |
| Score = a[PSA/H3.1-nucleosomes] +b[age] | 82% | <0.06 |
| Score = a[IL-6] -b[IL-8] | 75% | <0.05 |
| Score = a[IL-6] -b[IL-8] +c[age] | 83% | <0.06 |
| Score = a[IL-6]/[IL-8] +c[age] | 78% | <0.07 |
| Score = a[PSA] + b[IL-6]/[IL-8] | 77% | <0.08 |
| Score = [IL-6]/[IL-8] | 71% | <0.02 |

We also derived models using results obtained for nucleosomes and interleukins determined in plasma samples for the discrimination of men with high risk prostate cancer from men with no cancer or low risk prostate cancer. An example model is of the form Score = a[IL-6] -b[IL-8] +c[H3.1-nucleosomes] +d[PSA] as shown in Figure 9 which had AUC = 92%.

In one embodiment, the measuring step comprises the use of an algorithm listed in Figure 9, Table 1 or Table 2. Methods for deriving models or algorithms such as those in Table 1 or Table 2 are well known in the art and suitable software packages are available. Typical software tools for this purpose include SPSS (Statistical Package for the Social Sciences) and "R". These software packages provide for linear and non-linear data modelling of clinical data.

It will be clear to those skilled in the art, that any combination of the biomarkers disclosed herein may be used in panels and algorithms for the detection of prostate cancer, in particular high grade PCa, and that further markers may be added to a panel including these markers. It will also be clear any algorithms involving ratios can also use the inverse ratios *(i.e.* [A]/[B] or [B]/[A]). The p-values associated with the models were significant and would be expected to be even lower in a larger study size.

In one embodiment, Interleukin-8 is measured. In a further embodiment, the Interleukin-8 measurement additionally comprises determining a clinical parameter, such as age. In another embodiment, the Interleukin-8 measurement additionally comprises measuring the level of PSA. In one embodiment, Interleukin-8 and PSA are measured and the combined measurement takes the form of a ratio of, or the difference in, Interleukin-8 and PSA levels.

In another embodiment, the Interleukin-8 measurement additionally comprises measuring the level of PSA and nucleosomes or a component thereof, such as the level of H3.1 and/or H1. In one embodiment, PSA and nucleosomes are measured and the combined measurement takes the form of a ratio of, or the difference in, PSA and nucleosome levels. In a further embodiment, the combined measurement of Interleukin-8, PSA and nucleosomes additionally comprises determining a clinical parameter, such as age.

In one embodiment, Interleukin-6 is measured. In a further embodiment, the Interleukin-6 measurement additionally comprises determining a clinical parameter, such as age. In another embodiment, the Interleukin-6 measurement additionally comprises measuring the level of PSA. In one embodiment, Interleukin-6 and PSA are measured and the combined measurement takes the form of a ratio of, or the difference in, Interleukin-6 and PSA levels.

In another embodiment, the Interleukin-6 measurement additionally comprises measuring the level of PSA and nucleosomes or a component thereof, such as the level of H3.1 and/or H1. In one embodiment, PSA and nucleosomes are measured and the combined measurement takes the form of a ratio of, or the difference in, PSA and nucleosome levels. In a further embodiment, the combined measurement of Interleukin-6, PSA and nucleosomes additionally comprises determining a clinical parameter, such as age.

In one embodiment, Interleukin-6 and Interleukin-8 are measured and the combined measurement takes the form of a ratio of, or the difference in, Interleukin-6 and Interleukin-8 levels. In a further embodiment, the combined measurement of Interleukin-6 and Interleukin-8 additionally comprises determining a clinical parameter, such as age. In another embodiment, PSA, Interleukin-6 and Interleukin-8 are measured.

In one embodiment, PSA, Interleukin-6, Interleukin-8 and nucleosomes or a component thereof, such as H1, are measured. In one embodiment, PSA and nucleosomes are measured and the combined measurement takes the form of a ratio of, or the difference in, PSA and nucleosome levels.

Disclosed but not claimed is the use of a binding agent in the manufacture of a kit for use in a method of diagnosing prostate cancer in a body fluid sample, wherein said binding agents is specific for IL-8. The method comprises using the binding agent to detect or measure the level of IL-8 in a body fluid sample obtained from the patient, wherein a lower level of IL-8 compared to a control is indicative of the presence of prostate cancer. The use may additionally comprise binding agents specific for IL-6, PSA and/or nucleosomes or a component thereof, as described herein.

In one embodiment, the level of Interleukin-8 is measured as one of a panel of measurements including PSA and/or Interleukin-6 and/or nucleosomes or a component thereof. In a further embodiment, the panel of measurements includes Interleukin-8 and one or more of PSA, Interleukin-6, histone H1 and histone H3.1

Treatments available for PCa include surgery, radiotherapy including brachytherapy, hormone therapy including surgical and medical castration, as well as a variety of drug treatments.

In one embodiment, the prostate cancer is high grade prostate cancer. References to "high grade" refer to prostate cancer with a Gleason score of 7 (4+3) or above. They may also be referred to as "poorly differentiated" or "aggressive" prostate cancers.

According to an aspect of the invention there is provided the use of a panel test to detect a patient with high grade prostate cancer (PCa), wherein the panel test comprises reagents to detect measurements of PSA, Interleukin-6 (IL-6), Interleukin-8 (IL-8) and nucleosomes or a component thereof. In one embodiment, the panel test is for use with a blood, serum or plasma sample obtained from the patient.

In one embodiment, the control comprises a healthy subject, a non-diseased subject and/or a subject without cancer. In one embodiment, the control comprises a subject with low grade prostate cancer. In one embodiment, the method comprises comparing the amount of biomarker(s) present in a blood, serum or plasma sample obtained from the subject with the amount of biomarker(s) present in a blood, serum or plasma sample obtained from a normal subject. It will be understood that a "normal" subject refers to a healthy/non-diseased subject.

According to a further aspect of the invention there is provided a method of identifying a patient in need of a prostate biopsy comprising applying a blood, serum or plasma sample obtained from a patient to a panel test as defined herein, and using the results obtained from the panel test to identify whether the patient is in need of a prostate biopsy.

In one embodiment, the method described herein is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

Thus, the invention also provides a method of monitoring efficacy of a therapy for a disease state in a subject, suspected of having such a disease, comprising detecting and/or quantifying the biomarker present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the nature or amount of the biomarker(s) in test samples taken on different occasions.

Thus, according to a further aspect of the invention, there is provided a method for monitoring efficacy of therapy for a disease state in a human or animal subject, comprising:
(a) quantifying the amount of the biomarker as defined herein; and
(b) comparing the amount of said biomarker in a test sample with the amount present in one or more control(s) and/or one or more previous test sample(s) taken at an earlier time from the same test subject.

A change in the level of the biomarker in the test sample relative to the level in a previous test sample taken earlier from the same test subject may be indicative of a beneficial effect, e.g. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances. In a further example the monitoring of more rapid changes due to fast acting therapies may be conducted at shorter intervals of hours or days.

According to a further aspect of the invention there is provided the use of the panel test as defined herein to identify a patient in need of treatment for PCa.

According to a further aspect there is provided the use of the panel test as defined herein to monitor a patient for progression of PCa disease. Examples of this aspect include use to detect disease progression in watchful waiting, active surveillance and monitoring post-surgery or other treatment for relapse.

According to a further aspect there is provided the use of the panel test (or methods of the invention) as defined herein for pre-treatment risk stratification of a prostate cancer patient (Humphrey; 2014 and Barry and Nelson; 2015). Reference to "risk stratification" refers to the process of separating patients into different groups depending on their risk, e.g. categorising a patient as high risk, intermediate risk or low risk. Risk groups may be further
refined, for example the intermediate risk group may be refined to intermediate favourable and intermediate unfavourable risk groups (NCCN; 2018). The invention described herein may be used to determine whether a patient is high risk and therefore requires treatment for prostate cancer (e.g. see Example 3 and Example 5). It is clear that the methods of the invention are superior to the use of PSA measurements alone and may be used in addition to, or in place of, PSA measurements in risk stratification criteria.

According to a further aspect there is provided the use of the panel test as defined herein to evaluate the effectiveness of a PCa treatment in a patient.

In that aspect, the panel test comprises (optionally among others) reagents to detect PSA and IL-8. IL-8 levels are lower in patients with higher grade Gleason scores *(i.e.* high grade prostate cancer), for example grade 7(4+3) and above, such as grade 8, grade 9 or grade 10. Example algorithms for interpretation of the results may be of the form: Panel Score = a[PSA] - b[IL-8] or Panel Score = a[PSA]/[IL-8].

In an example, the panel test comprises (optionally among others) reagents to detect IL-6 and IL-8. IL-8 levels are lower in patients with higher grade Gleason scores *(i.e.* high grade prostate cancer), for example grade 7(4+3) and above, such as grade 8, grade 9 or grade 10. An example algorithm for interpretation of the results may be of the form: Panel Score = a[IL-6] - b[IL-8] or Panel Score = a[IL-6]/[IL-8].

In a further embodiment, a PSA measurement is used in combination with an Interleukin-8 level measurement in blood, serum or plasma as a biomarker for prostate cancer. In a further embodiment, a PSA measurement is used in combination with an Interleukin-8 level measurement and an Interleukin-6 measurement and a nucleosome level measurement in blood, serum or plasma as a biomarker for prostate cancer. The combined measurement may take the form of a ratio of circulating PSA/Interleukin levels or a ratio of circulating PSA/nucleosome levels. It will be clear to those skilled in the art that any nucleosome measurement may be used in this way including nucleosomes *per se* (*i.e.* total nucleosome level), as well as any subset of nucleosomes with particular structures and/or epigenetic features as described herein.

In a preferred embodiment, the panel test comprises reagents to detect PSA, nucleosomes or a component thereof, IL-6 and IL-8. An example algorithm for interpretation of the results may be of the form: Panel Score=a[IL-6]-b[IL-8] +c[PSA/H3.1-nucleosomes]+d[PSA/H1-nucleosomes]. Another example algorithm for interpretation of the results may be of the form: Panel Score=a[IL-6]-b[IL-8]+c[H3.1-nucleosomes]+d[PSA]. The negative value of the coefficient "b" pertaining to the IL-8 level pertains to the lower level that occurs in higher grade or higher risk disease.

In another embodiment of the disclosure, the panel test comprises reagents to detect PSA, nucleosomes or a component thereof and IL-8. An example algorithm for interpretation of the results may be of the form: Panel Score=-a[IL-8] +b[PSA/H3.1-nucleosomes]+c[PSA/H1-nucleosomes].

In other embodiments the panel may include reagents to detect total nucleosome levels and/or H1-nucleosome levels and/or may include other nucleosome measurements. Some further examples are listed as example algorithm forms in **Table 1.**

In another embodiment, IL-8 is included as a parameter in a prostate cancer risk stratification system.

We have found that age is an independent predictor of high grade PCa. It has also been reported that PSA levels are age dependent (Heidegger et al; 2015). It is therefore possible to combine age with other algorithms in a graphical format as shown for the algorithm score = a[IL-6] -b[IL-8] +c[PSA/H3.1-nucleosomes] +d[PSA/H1-nucleosomes] in **Figure** 7. One advantage of this method is that it can be used to refine the algorithm to isolate an age/algorithm area of the graph in which all or most of the subjects with a given condition occur (positive selection for a rule-in test), or in which there are very few or no individual subjects with a given condition (negative selection for a rule-out test).

In an embodiment of the invention, an age dependent cut-off is used for the output of an algorithm. The variable cut-off used to categorise a sample as positive or negative may be varied based on age in days, months, years or any other unit of time. For example, the cut-off might take a value of [a] at 70 years, [b] at 71, [c] at 72 etc. In one embodiment the age dependent cut-off is included in the algorithm.

References to "subject" or "patient" are used interchangeably herein. In one embodiment, the patient is a human patient. The use, panels and methods described herein may be performed *in vitro* or *ex vivo.*

In one example, said detection or measurement comprises an immunoassay, immunochemical, mass spectroscopy, chromatographic, chromatin immunoprecipitation or biosensor method.

In one example, the detection or measurement comprises an immunoassay. In a preferred example there is provided a 2-site immunoassay method. In particular, such a method is preferred for the measurement of nucleosome incorporated epigenetic features *in situ* employing an immobilized anti-nucleosome binding agent in combination with a labelled anti-histone modification or anti-histone variant or anti-DNA modification or anti-adducted protein detection binding agent. In another example, there is provided a 2-site immunoassay employing a labelled anti-nucleosome detection binding agent in combination with an immobilized anti-histone modification or anti-histone variant or anti-DNA modification or anti-adducted protein binding agent.

Detecting or measuring the level of the biomarker(s) may be performed using one or more reagents, such as a suitable binding agent. In one embodiment, the one or more binding agents comprises a ligand or binder specific for the desired biomarker, e.g. IL-8, IL-6, PSA, nucleosomes or component part thereof (such as an epigenetic feature of a nucleosome), or a structural/shape mimic of the nucleosome or component part thereof.

It will be clear to those skilled in the art that the terms "antibody", "binder" or "ligand" in regard to any aspect of the invention is not limiting but intended to include any binder capable of binding to particular molecules or entities and that any suitable binder can be used in the method of the invention. It will also be clear that the term "nucleosomes" is intended to include mononucleosomes and oligonucleosomes and any such chromatin fragments that can be analysed in fluid media.

Methods of detecting biomarkers are known in the art. In one example, the reagents comprise one or more ligands or binders. In one example, the ligands or binders include naturally occurring or chemically synthesised compounds, capable of specific binding to the desired target. A ligand or binder may comprise a peptide, an antibody or a fragment thereof, or a synthetic ligand such as a plastic antibody, or an aptamer or oligonucleotide, capable of specific binding to the desired target. The antibody can be a monoclonal antibody or a fragment thereof. A ligand may be labelled with a detectable marker, such as a luminescent, fluorescent, enzyme or radioactive marker; alternatively or additionally a ligand according to the invention may be labelled with an affinity tag, e.g. a biotin, avidin, streptavidin or His (e.g. hexa-His) tag. Alternatively, ligand binding may be determined using a label-free technology for example that of ForteBio Inc.

Diagnostic or monitoring kits (or panels) are provided for performing methods of the invention. Such kits will suitably comprise one or more ligands for detection and/or quantification of the biomarker according to the invention, and/or a biosensor, and/or an array as described herein, optionally together with instructions for use of the kit.

Disclosed but not claimed is a kit for detecting the presence of a disease state, comprising a biosensor capable of detecting and/or quantifying one or more of the biomarkers as defined herein. As used herein, the term "biosensor" means anything capable of detecting the presence of the biomarker. Examples of biosensors are described herein. Biosensors may comprise a ligand binder or ligands, as described herein, capable of specific binding to the biomarker. Such biosensors are useful in detecting and/or quantifying a biomarker of the invention.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, e.g. in the ward, outsubjects' department, surgery, home, field and workplace. Biosensors to detect one or more biomarkers of the invention include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers determined in the uses and methods of the invention.

Biomarkers for detecting the presence of a disease are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers determined in the uses and methods of the invention can be employed in methods for screening for compounds that modulate the activity of the biomarker.

Thus, in a further aspect, there is provided the use of a binder or ligand, as described, which can be a peptide, antibody or fragment thereof or aptamer or oligonucleotide directed to a biomarker; or the use of a biosensor, or an array, or a kit, to identify a substance capable of promoting and/or of suppressing the generation of the biomarker.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying subjects most likely to respond to a particular therapeutic treatment, drug screening and development. Biomarkers and uses thereof are valuable for identification of new drug treatments and for discovery of new targets for drug treatment.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, *i.e.* for drug screening and drug development.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, *e.g.* a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

For example, detecting and/or quantifying can be performed using an immunological method, such as an immunoassay. Immunoassays include any method employing one or more antibodies or other specific binders directed to bind to the biomarkers defined herein. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame et al. (1997) and van Nieuwenhuijze et al. (2003).

In another example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT^{®} (Applied Biosystems, CA, USA), or iTRAQ^{®} (Applied Biosystems, CA, USA). Liquid chromatography (*e.g.* high pressure liquid chromatography (HPLC) or low pressure liquid chromatography (LPLC)), thin-layer chromatography, NMR (nuclear magnetic resonance) spectroscopy could also be used.

Methods involving identification and/or quantification of one or more biomarkers can be performed on bench-top instruments, or can be incorporated onto disposable, diagnostic or monitoring platforms that can be used in a non-laboratory environment, e.g. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

The identification of biomarkers for a disease state permits integration of diagnostic procedures and therapeutic regimes. Detection of a biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker of the invention, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

It will be understood that the embodiments described herein may be applied to all aspects of the invention, *i.e.* the embodiment described for the uses may equally apply to the claimed methods and so forth. The scope of protection is defined by the appended claims.

The invention will now be illustrated with reference to the following non-limiting examples.

### EXAMPLES

### Example 1

Venous blood samples were taken from 84 men referred for prostate biopsy to investigate suspected PCa. The whole blood was added to a standard gel SST (serum separator tube) and left to clot for 30-60 minutes before centrifugation. The isolated serum fraction was transferred to a standard cryotube and frozen until assayed.

All the men underwent a prostate biopsy procedure at which at least 12 needle core samples of prostate tissue were removed. Microscopic examination of the tissue samples was performed to check for the presence of abnormal tissue. No abnormal cancer tissue was observed in 35 men who were diagnosed as having no cancer (negative biopsy result). Abnormal cancer tissue was observed in 49 men (positive biopsy result) who were diagnosed with PCa and the prostate tissue samples of these men were classified according to Gleason grade system known in the art, and as described earlier. Of the 49 men with a positive PCa biopsy result, 16 men were classified with Gleason grade 6, 17 men with grade 7(3+4), 6 men with grade 7(4+3), 3 men with grade 8, and 7 men with grade 9.

We measured serum levels of PSA, nucleosomes containing histone H3.1, nucleosomes containing histone H1, IL-6 and IL-8 in blood samples taken from the men. PSA, IL-6 and IL-8 were measured using commercially available immunoassay methods. H3.1-nucleosomes were measured using an ELISA employing one antibody directed to bind to histone H3.1 and one antibody directed to bind to an epitope present in intact nucleosomes. Nucleosomes containing histone H1 were measured using an ELISA employing one antibody directed to bind to histone H1 and one antibody directed to bind to an epitope present in intact nucleosomes.

The vast majority of the men referred for biopsy had elevated PSA levels including those subsequently found to have no cancer on biopsy and PSA measurements alone were a poor discriminator for high grade cancers **(****Figure 1** and **2****).**

When the markers were combined into marker panels and interpreted using algorithms, extremely high discrimination was found. The models/algorithms were derived using the statistical programming language R (R Core Team; 2017). Logistic Regression (LR) was conducted, using the R function Irm, from the package rms (Harrell Jr; 2018), on combinations of assay results, patient variables and clinical parameters. Receiver Operator Characteristic (ROC) curves were calculated using the R function pROC (Robin *et al;* 2011).

The models produced by the program were tested for statistical significance (p-values) for the whole model as well as the constituent terms and by cross-validation in which the model is checked for robustness on randomly selected data subsets. Some models/algorithms developed, together with their AUC for the discrimination of men with high grade PCa from all others *(i.e.* men with low grade PCa or no cancer) are shown in Table 2. A constant term may optionally be used to move the cut-off point to a convenient value (e.g. zero).

**Table 2. Example algorithms with observed AUC and p-values**

| Panel and Algorithm | AUC | p |
|---|---|---|
| Score = 0.49[IL-6] -0.14[IL-8] +0.061[PSA/H3.1-nucleosomes] -0.027[PSA/H1-nucleosomes] | 94% | <0.01 |
| Score = -0.14[IL-8] +0.016[PSA/H3.1-nucleosomes] -0.028[PSA/H1-nucleosomes] | 89% | <0.05 |
| Score = 0.37[IL-6] +0.02[PSA/H3.1-nucleosomes] -0.049[PSA/H1-nucleosomes] | 87% | <0.01 |
| Score = 0.093[age] +0.012[PSA/H3.1-nucleosomes] - 0.026[PSA/H1-nucleosomes] | 87% | <0.05 |
| Score = 0.13[age] - 0.10[IL-8] + 0.0032[PSA/H3.1-nucleosomes] | 85% | <0.02 |
| Score = - 0.105[IL-8] + 0.0094 [PSA/H1-nucleosomes] | 81% | <0.03 |
| Score = 0.044[PSA] -0.072[IL-8] - 0.30 | 81% | <0.04 |
| Score = [PSA/IL-8] | 79% | <0.02 |
| Score = 0.015[PSA/H3.1-nucleosomes] - 0.034[PSA/H1-nucleosomes] | 79% | <0.03 |
| Score = - 0.078[IL-8] + 0.0042[PSA/H3.1-nucleosomes] | 79% | <0.02 |
| Score = 0.12 [age] - 0.074[IL-8] | 79% | <0.03 |
| Score = [PSA/H3.1-nucleosomes] | 77% | <0.02 |
| Score = 0.0025[PSA/H3.1-nucleosomes] + 0.10 [age] | 82% | <0.06 |
| Score = 0.24[IL-6] - 0.061[IL-8] | 75% | <0.05 |
| Score = 0.28[IL-6] - 0.088[IL-8] + 0.12[age] | 83% | <0.06 |
| Score = 5.8[IL-6]/[IL-8] + 0.11[age] | 78% | <0.07 |
| Score = 0.032[PSA] -0.053[IL-8]/[IL-6] | 77% | <0.08 |
| Score = [IL-8]/[IL-6] | 71% | <0.02 |

The results can be explained as follows:
Median PSA levels were higher in men with PCa of Gleason grade 8 or 9 than in men with low grade cancer or no cancer, but men with PCa of grade 7(4+3) were indiscernible from those with low grade cancer. Overall, PSA alone identified 33% of men with high grade PCa from all others at 90% specificity with AUC=74%. However, less than 20% of men with no cancer or low grade cancer could be identified by low PSA levels alone as not in need of a prostate biopsy without missing any high grade cancers **(****Figure 1** and **2****).**

Similarly, we found that median IL-6 levels were higher in men with PCa of Gleason grade 8 or 9 than in men with low grade cancer or no cancer, but men with PCa of grade 7(4+3) were again indiscernible. The overall sensitivity for high grade PCa was 17% at 90% specificity with AUC=59%. Less than 3% of men with no cancer or low grade cancer could be identified by low IL-6 levels alone as not in need of a prostate biopsy without missing any high grade cancers.

In contrast, we found IL-8 levels were lower in men with high grade PCa (Gleason grade of 7(4+3) or above) than in men with low grade cancer or no cancer. The overall sensitivity for high grade PCa observed was 33% at 90% specificity with AUC=62% and we observed a general decrease in IL-8 levels with Gleason grade **(****Figure 3****).** Less than 10% of men with no cancer or low grade cancer could be identified by low IL-8 levels alone as not in need of a prostate biopsy without missing any high grade cancers.

When IL-6 and IL-8 levels were combined using an algorithm in the form of a Panel Score=a[IL-6]-b[IL-8], we observed a sensitivity for high grade PCa of 33% at 90% specificity with AUC=74%. Almost 50% of men with no cancer or low grade cancer could be identified as not in need of a prostate biopsy whilst missing 5% of high grade cancers. This shows that a diagnostic algorithm designed around an increase of IL-6 levels combined with a decline of IL-8 levels in PCa patients gives a more accurate result than the PSA test which is the most used current test. Embodiments may include the form of the difference in IL-6 and IL-8 levels, as described above, or a ratio of IL-6 and IL-8 levels.

When PSA levels and IL-8 levels were combined using an algorithm of the form Panel Score=a[PSA]-b[IL-8], we observed a sensitivity for high grade PCa of 72% at 90% specificity with AUC=81% and an improved stepwise increase in algorithm result with Gleason grade **(****Figure 4****).** Similarly, an algorithm involving a ratio of PSA/IL-8 levels gave a sensitivity for high grade PCa of 50% at 90% specificity with AUC=79%. Thus, combining PSA and IL-8 measurements in an algorithm greatly improves diagnostic accuracy over PSA alone. Embodiments may include the form of the difference in PSA and IL-8 levels or a ratio of PSA and IL-8 levels.

The observed individual levels of nucleosomes containing histone H3.1 and nucleosomes containing histone H1 were not greatly changed with high grade PCa. However, when PSA levels were combined with levels of nucleosomes containing histone H3.1 using an algorithm of the form of a ratio as Panel Score=a[PSA/H3.1-nucleosomes], we observed a sensitivity for high grade PCa of 39% at 90% specificity with AUC=79%. Moreover, almost 50% of men with no cancer or low grade cancer could be identified as not in need of a prostate biopsy whilst missing <6% of high grade cancers. We also observed an improved stepwise increase in ratio levels with Gleason grade **(****Figure 5****).** Thus, combining the PSA and H3.1-nucleosome measurements improves diagnostic accuracy over PSA alone. Embodiments may include the form of a ratio of PSA and IL-8 levels or the difference in PSA and IL-8 levels.

When PSA levels were combined with levels of nucleosomes containing histone H1 using an algorithm of the form Panel Score=a[PSA/H1-nucleosomes], we observed a sensitivity for high grade PCa of 25% at 90% specificity with AUC=73% . Almost 30% of men with no cancer or low grade cancer could be identified as not in need of a prostate biopsy whilst missing no high grade cancers.

When PSA levels were combined with IL-6, levels of nucleosomes containing histone H3.1 levels and levels of nucleosomes containing histone H1 using an algorithm of the form Panel Score=a[IL-6] +b[PSA/H3.1-nucleosomes]+c[PSA/H1-nucleosomes], we observed a sensitivity for high grade PCa of 63% at 90% specificity with AUC=87%. Despite the small size of the study the algorithm was statistically significant and all p-values associated with the model and each of its component parts were statistically significant (p<0.005).

When PSA levels were combined with IL-8, levels of nucleosomes containing histone H3.1 and levels of nucleosomes containing histone H1 using an algorithm of the form Panel Score=-a[IL-8] +b[PSA/H3.1-nucleosomes]+c[PSA/H1-nucleosomes], we observed a sensitivity for high grade PCa of 88% at 89.7% specificity with AUC=89%. Using this algorithm, about 25% of men with no cancer or low grade cancer could be identified as not in need of a prostate biopsy whilst missing no high grade cancers. Despite the small size of the study the algorithm was statistically significant and all p-values associated with the model and each of its component parts were statistically significant (p<0.05).

When PSA levels were combined with levels of IL-6, IL-8, nucleosomes containing histone H3.1 and nucleosomes containing histone H1 using an algorithm of the form Panel Score=a[IL-6]-b[IL-8] +c[PSA/H3.1-nucleosomes]+d[PSA/H1-nucleosomes], we observed a sensitivity for high grade PCa of 94% at 88% specificity with AUC=94%. Using this algorithm, 45% of men with no cancer or low grade cancer could be identified as not in need of a prostate biopsy whilst missing no high grade cancers. Moreover, this model gave a clear stepwise increase in median results with Gleason grade **(****Figure** 6). All p-values associated with the model and all its component terms were statistically significant (the highest of any p-value being p<0.007) and the model was robust to cross-validation analysis.

### Example 2

The most accurate model in Example 1 was able to rule out high grade PCa *(i.e.* to identify men not in need of immediate treatment) in 45% of men with no cancer or low grade cancer whilst missing no high grade cancers. This could be improved by the use of an age dependent cut-off for the algorithm such that a constant cut-off was used for men of approximate age ≤67 with a decreasing cut-off in men of age >67 such the cut-off was reduced by approximately 0.7 for each year of age above 67. The results are presented in **Figure 7****.** The lower left area of the graph in **Figure 7** excludes subjects with an algorithm score above an age-dependent cut-off. The result is that the lower left area of the graph in **Figure 7** includes no individuals with high grade cancer and could be used to identify individuals who do not have high grade disease with a high negative predictive value. In the example shown here >70% of subjects with no cancer and >40% of subjects with low grade cancer can be predicted to be negative for high grade cancer with zero false negatives.

### Example 3

The subjects with PCa included in Example 1 who were allocated Gleason Grade Scores were subsequently categorised as low risk, intermediate risk or high risk according to recognised pre-treatment risk stratification criteria including PSA level, Gleason Score and clinical stage below (Humphrey; 2014 and Barry and Nelson; 2015).

**Table 3. Categorisation of PCa subjects**

| **Risk Group** | **Clinical Stage** | **Gleason Score** | **Serum PSA** |
|---|---|---|---|
| **Low Risk** | ≤T2a | ≤6 | <10ng/ml |
| **Intermediate Risk** | One elevated risk factor: | | |
| | Clinical stage ≥T2a disease, Gleason score ≥7, PSA ≥10 ng/mL | | |
| **High Risk** | Two elevated risk factors | | |

In this risk system, high risk patients should be treated and low risk patients should be monitored. The decision on treatment of patients with intermediate risk lies with the clinician and the patient but they should at least be put under active surveillance and are likely to receive hormone therapy.

We reapplied the algorithms derived by modelling Gleason Scores on the risk classified patients in Example 1 on the risk stratified data to discriminate men at high risk from those with low risk or no cancer. Serum PSA alone was a better model of risk stratification than Gleason score which is to be expected as it forms part of the risk stratification process. Serum PSA discriminated high risk patients from those with low risk or no cancer with an AUC of 84% and a sensitivity of 80% at 85% specificity. Addition of IL-8 as a marker in a ratio algorithm score = [PSA]/[IL-8] increased the AUC to 87%, gave a sensitivity of 80% at 85% specificity and was able to discriminate 40% of men who are low risk or do not have cancer with no false negative results for men with high risk PCa *(i.e.* no men with high risk cancer were "missed"). Application of the algorithm "Score = -0.14[IL-8] +0.016[PSA/H3.1-nucleosomes] - 0.028[PSA/H1-nucleosomes]" to the risk stratified data gave an AUC of 86% and a sensitivity of 84% at 93% specificity. Thus, the use of interleukin and nucleosome markers greatly improves on the performance of PSA alone, even to predict the outcome of risk stratification procedures that include PSA.

We then reapplied the algorithms derived by modelling Gleason Scores on the risk classified patients in Example 1 on the risk stratified data to discriminate men at high risk or intermediate risk from those with low risk or no cancer. Again, serum PSA alone was a better model of risk stratification than Gleason score which is to be expected as it forms part of the risk stratification process. Serum PSA discriminated high risk or intermediate risk patients from those with low risk or no cancer with an AUC of 69% and a sensitivity of 36% at 90% specificity. Addition of IL-8 as a marker in the ratio algorithm "score = [PSA]/[IL-8]" increased the AUC to 72%, gave a sensitivity of 44% at 90% specificity and was able to discriminate 40% of men who are low risk or do not have cancer with 15% false negative results for men with high or intermediate risk PCa, but the previous results above showed a zero miss rate for men with high risk PCa so these misclassified men are presumably intermediate risk. Application of the algorithm "score = 0.49[IL-6] -0.14[IL-8] +0.061[PSA/H3.1-nucleosomes] -0.027[PSA/H1-nucleosomes]" to the risk stratified data gave an AUC of 75% and a sensitivity of 52% at 92% specificity. Again, the use of interleukin and nucleosome markers greatly improves on the performance of PSA alone, even to predict the outcome of risk stratification procedures that include PSA.

These data indicate that, as well as predicting the grade of cancer found on biopsy, the methods of the present invention can predict the outcome of the combined risk stratification process for the patient. It is clear therefore that the method of the invention has uses in avoiding unnecessary biopsies and may also be used to improve disease outcomes by improved risk stratification of prostate cancer patients by inclusion of the methods in the risk stratification process.

### Example 4

EDTA plasma samples were taken from the same 84 men referred for prostate biopsy to investigate suspected PCa described in Example 1. The serum and plasma samples were taken at the same time. The isolated plasma fraction was transferred to a standard cryotube and frozen until assayed.

We measured plasma levels of H3.1-nucleosomes, IL-6 and IL-8 in blood samples taken from the men. The levels of these markers were combined with serum PSA levels into marker panels and interpreted using algorithms that compared the results with disease Gleason grade. **Figure 8** shows the results of a regression analysis algorithm of the form "Panel Score = 0.80[IL-6] -0.25[IL-8] +0.79[H3.1-nucleosomes] +0.044[PSA]" with Gleason grade determined on prostate biopsy. It is clear that men with high grade disease can be differentiated from those with low grade disease or no cancer. Moreover, almost 70% of men with low grade disease or no cancer can be identified without a single false negative misclassification of any man with high grade disease. This method of the invention may therefore be used to avoid unnecessary biopsies.

### Example 5

The results for the EDTA plasma samples described in Example 4 were combined with serum PSA levels into marker panels and interpreted using algorithms that compared the results with disease risk stratification. Figure 9 shows the results of a regression analysis algorithm of the form "Panel Score = 0.603[IL-6] -0.105[IL-8] +0.892[H3.1-nucleosomes] +0.080[PSA]" with disease risk stratification (low risk, intermediate risk favourable, intermediate risk unfavourable or high risk cancer) determined on prostate biopsy. The algorithm was developed as a regression equation to optimise the discrimination of men with high risk PCa from those men with low risk disease or no cancer. The results correlate with clinical risk category and median levels increase stepwise with increasing risk. The discrimination of the algorithm for men subsequently diagnosed with High Risk PCa from other men referred for biopsy with Low Risk PCa or no cancer is shown in the ROC curve in Figure 9 (AUC=92%). The assay panel and algorithm detected 65% of men with high risk PCa at 96% specificity. Moreover, 74% of men who did not have high risk PCa were identified as negative for high risk PCa with zero false negative results (100% detection of High Risk PCa). The algorithm classified 54% of intermediate favourable risk patients and 38% of intermediate unfavourable risk patients as low risk. Thus, ruling out biopsy on this basis would mean some men with intermediate risk disease would be monitored rather than treated. However, this is the usual clinical outcome for these men in any event.

For comparison, the PSA test detected 42% of men with high risk disease at 96% specificity and identified 22% of men who did not have high risk PCa with zero false negative results as shown in Figure 10.

The blood test of the invention gives more accurate information relating to the clinical risk of the patient than the PSA test and can be used to stratify patients, to triage those patients most in need of treatment, to monitor patients for increasing risk category over time with repeated testing and to avoid unnecessary prostate biopsies.

### REFERENCES

Barry & Nelson, J Urol, 194: 1534, 2015
Batra et al, Journal of Biomarkers, 2014: 1-12, 2014
Chadha et al, Clin Cancer Investig J, 3: 72-79, 2014
Crawford et al, Cancernetwork, 28(2), 2014
Cuchiarra et al, Eur Urol, 73: 572-582, 2018
Du et al, Cancer Chemother Pharmacol, 81: 1111-1119, 2018
Filella et al, Pharmacogenomics Pers Med, 11: 83-94, 2018
Harrell Jr, rms: Regression Modeling Strategies. R package version 5.1-2. https://CRAN.R-project.org/package=rms, 2018
Heidegger et al, PLoS ONE, 10(7): e0134134, 2015
Herranz and Esteller, Methods Mol Biol, 361: 25-62, 2007
Holdenrieder et al, Int J Cancer, 95: 114-20, 2001
Holdenrieder & Stieber, Crit Rev Clin Lab Sci, 46(1): 1-24, 2009
Humphrey. Cancers of the male reproductive organs. In: World Cancer Report, World Health Organization, Lyon, 2014
Komatsu et al, Cancer, 118(12): 3208-3221, 2012
Madej-Michniewicz et al, Nature Scientific Reports, 5: 14382, 2015
Martin et al, JAMA, 319: 883-895, 2018
NCCN Clinical Practice Guidelines in Oncology. Prostate Cancer. Version 4, 2018
R Core Team (2017). R: A language and environment for statistical computing.
R Foundation for Statistical Computing. https://www.R-project.org/, 2017
Robin et al, BMC Bioinformatics, 12: 77, 2017
Salgame et al, Nucleic Acids Res, 25(3): 680-681, 1997
Singh and Lokeshwar, Mol Cancer, 8: 57, 2009
Stephan et al, Clin Chem, 59: 306-314, 2013
Taniguchi and Karin, Semin Immunol, 26: 54-74, 2014
van Nieuwenhuijze et al, Ann Rheum Dis, 62: 10-14, 2003
Veltri et al, Urology, 53: 139-47, 1999
Wang and Sun, Int J Oncology, 44: 1032-1040, 2014
Xia et al, PLoS ONE, 10(4): e0123484, 2015
Xie, Cytokine Growth Factor Rev, 12: 375-391, 2001
Xu et al, Cancer Res, 69: 3267-3271, 2009
Xu et al, PLoS ONE, 7(3): e32905, 2012

## Claims

1. Use of Interleukin-8 as a biomarker in a blood, serum or plasma sample for diagnosing and/or monitoring prostate cancer, wherein a lower level of Interleukin-8 compared to a control is indicative of the presence and/or progression of prostate cancer.

2. The use as defined in claim 1, which additionally comprises Interleukin-6 as a biomarker, wherein a higher level of Interleukin-6 compared to a control is indicative of the presence and/or progression of prostate cancer.

3. The use as defined in claim 2, for diagnosing and/or monitoring prostate cancer, which additionally comprises one or more biomarkers selected from the list consisting of: Prostate Specific Antigen (PSA) and nucleosomes or a component thereof.

4. The use as defined in claim 3, wherein the component of a nucleosome is an epigenetic feature of a nucleosome, preferably wherein the epigenetic feature of a nucleosome is histone H3.1.

5. The use as defined in any one of claims 1 to 4, for diagnosing the grade of prostate cancer, preferably wherein the grade of prostate cancer is high grade prostate cancer.

6. The use as defined in any one of claims 1 to 5, for risk stratification of a patient with suspected prostate cancer.

7. A method of diagnosing prostate cancer in a patient, comprising:
detecting or measuring the level of Interleukin-8, optionally in combination with at least one biomarker selected from the list consisting of: PSA and nucleosomes or a component thereof, in a blood, serum or plasma sample obtained from the patient,
wherein a lower level of Interleukin-8 compared to a control is indicative of the presence of prostate cancer.

8. A method for assessing the suitability of a patient for a prostate biopsy, comprising:
detecting or measuring the level of Interleukin-8, optionally in combination with at least one biomarker selected from the list consisting of: PSA and nucleosomes or a component thereof, in a blood, serum or plasma sample obtained from the patient; and
wherein a lower level of Interleukin-8 compared to a control is indicative that the patient requires a prostate biopsy.

9. The method as defined in claim 7 or claim 8, which additionally comprises detecting or measuring the level of Interleukin-6, wherein a higher level of Interleukin-6 compared to a control is indicative of the presence of prostate cancer or that the patient requires a prostrate biopsy.

10. The method as defined in any one of claims 7 to 9, which additionally comprises determining at least one clinical parameter for the patient, preferably wherein the clinical parameter is selected from: age, sex and body mass index (BMI).

11. The method as defined in any one of claims 7 to 10, wherein Interleukin-6 and Interleukin-8 are measured and wherein the combined measurement takes the form of a ratio of, or of the difference in, Interleukin-6 and Interleukin-8 levels.

12. Use of a panel comprising reagents to detect Interleukin-8, Interleukin-6 and nucleosomes or a component thereof according to the use of any of claims 1 to 6 or any of the methods of claims 7-11.

13. Use of the panel as defined in claim 12, which comprises reagents to detect Interleukin-8, Interleukin-6, nucleosomes or a component thereof and PSA.

14. Use of the panel as defined in claim 12 or claim 13, wherein the component of a nucleosome is an epigenetic feature of a nucleosome, preferably wherein the epigenetic feature of a nucleosome is histone H3.1.

15. Use of the panel as defined in any one of claims 12 to 14, for detecting prostate cancer.

## Patentansprüche

1. Verwendung von Interleukin-8 als Biomarker in einer Blut-, Serum- oder Plasmaprobe zur Diagnose und/oder Überwachung von Prostatakrebs, wobei ein im Vergleich zu einer Kontrollgruppe niedrigerer Interleukin-8-Spiegel das Vorhandensein und/oder die Progression von Prostatakrebs angibt.

2. Verwendung nach Anspruch 1, die zusätzlich Interleukin-6 als Biomarker umfasst, wobei ein im Vergleich zu einer Kontrollgruppe höherer Interleukin-6-Spiegel das Vorhandensein und/oder die Progression von Prostatakrebs angibt.

3. Verwendung nach Anspruch 2 zur Diagnose und/oder Überwachung von Prostatakrebs, die zusätzlich ein oder mehrere Biomarker umfasst, ausgewählt aus der Liste, bestehend aus: prostataspezifischem Antigen (PSA) und Nukleosomen oder einer Komponente davon.

4. Verwendung nach Anspruch 3, wobei es sich bei der Komponente eines Nukleosoms um ein epigenetisches Merkmal eines Nukleosoms handelt, wobei es sich bei dem epigenetischen Merkmal eines Nukleosoms vorzugsweise um Histon H3.1 handelt.

5. Verwendung nach einem der Ansprüche 1 bis 4 zur Diagnose des Grades von Prostatakrebs, wobei es sich bei dem Grad von Prostatakrebs vorzugsweise um einen hochgradigen Prostatakrebs handelt.

6. Verwendung nach einem der Ansprüche 1 bis 5 zur Risikostratifizierung eines Patienten mit Verdacht auf Prostatakrebs.

7. Verfahren zum Diagnostizieren von Prostatakrebs bei einem Patienten, umfassend:
Nachweisen oder Messen des Interleukin-8-Spiegels, gegebenenfalls in Kombination mit mindestens einem Biomarker, ausgewählt aus der Liste, bestehend aus: PSA und Nukleosomen oder einer Komponente davon, in einer von dem Patienten erhaltenen Blut-, Serum- oder Plasmaprobe,
wobei ein im Vergleich zu einer Kontrollgruppe niedrigerer Interleukin-8-Spiegel das Vorhandensein von Prostatakrebs angibt.

8. Verfahren zum Beurteilen der Eignung eines Patienten für eine Prostatabiopsie, umfassend:
Nachweisen oder Messen des Interleukin-8-Spiegels, gegebenenfalls in Kombination mit mindestens einem Biomarker, ausgewählt aus der Liste, bestehend aus: PSA und Nukleosomen oder einer Komponente davon, in einer von dem Patienten erhaltenen Blut-, Serum- oder Plasmaprobe; und
wobei ein im Vergleich zu einer Kontrollgruppe niedrigerer Interleukin-8-Spiegel angibt, dass der Patient eine Prostatabiopsie benötigt.

9. Verfahren nach Anspruch 7 oder Anspruch 8, das zusätzlich Nachweisen oder Messen des Interleukin-6-Spiegels umfasst, wobei ein im Vergleich zu einer Kontrollgruppe höherer Interleukin-6-Spiegel das Vorhandensein von Prostatakrebs angibt oder angibt, dass der Patient eine Prostatabiopsie benötigt.

10. Verfahren nach einem der Ansprüche 7 bis 9, das zusätzliche Bestimmen mindestens eines klinischen Parameters für den Patienten umfasst, wobei der klinische Parameter vorzugsweise ausgewählt ist aus: Alter, Geschlecht und Body-Mass-Index (BMI).

11. Verfahren nach einem der Ansprüche 7 bis 10, wobei Interleukin-6 und Interleukin-8 gemessen werden und wobei die kombinierte Messung die Form eines Verhältnisses oder der Differenz von Interleukin-6- und Interleukin-8-Spiegel annimmt.

12. Verwendung eines Panels, umfassend Reagenzien zum Nachweisen von Interleukin-8, Interleukin-6 und Nukleosomen oder einer Komponente davon gemäß der Verwendung nach einem der Ansprüche 1 bis 6 oder einem der Verfahren nach Anspruch 7-11.

13. Verwendung des Panels nach Anspruch 12, das Reagenzien zum Nachweisen von Interleukin-8, Interleukin-6, Nukleosomen oder einer Komponente davon und PSA umfasst.

14. Verwendung des Panels nach Anspruch 12 oder Anspruch 13, wobei es sich bei der Komponente eines Nukleosoms um ein epigenetisches Merkmal eines Nukleosoms handelt, wobei es sich bei dem epigenetischen Merkmal eines Nukleosoms vorzugsweise um Histon H3.1 handelt.

15. Verwendung des Panels nach einem der Ansprüche 12 bis 14 zum Nachweisen von Prostatakrebs.

## Revendications

1. Utilisation de l'interleukine-8 en tant que biomarqueur dans un échantillon de sang, de sérum ou de plasma pour le diagnostic et/ou la surveillance du cancer de la prostate, dans lequel une diminution du taux d'interleukine-8 par rapport à un témoin indique la présence et/ou la progression du cancer de la prostate.

2. Utilisation telle que définie dans la revendication 1, qui comprend en outre l'interleukine-6 en tant que biomarqueur, dans laquelle un taux accru d'interleukine-6 par rapport à un témoin indique la présence et/ou la progression du cancer de la prostate.

3. Utilisation telle que définie dans la revendication 2, pour le diagnostic et/ou le suivi du cancer de la prostate, qui comprend en outre un ou plusieurs biomarqueurs choisis parmi la liste constituée : de l'antigène prostatique spécifique (PSA) et des nucléosomes ou d'un composant de ceux-ci.

4. Utilisation telle que définie dans la revendication 3, dans laquelle le composant d'un nucléosome est une caractéristique épigénétique d'un nucléosome, de préférence dans laquelle la caractéristique épigénétique d'un nucléosome est l'histone H3.1.

5. Utilisation telle que définie dans l'une quelconque des revendications 1 à 4, pour le diagnostic du grade du cancer de la prostate, de préférence dans laquelle le grade du cancer de la prostate est un cancer de la prostate de grade élevé.

6. Utilisation telle que définie dans l'une quelconque des revendications 1 à 5, pour la stratification du risque d'un patient suspecté d'être atteint d'un cancer de la prostate.

7. Procédé de diagnostic du cancer de la prostate chez un patient, comprenant :
la détection ou la mesure du taux d'interleukine-8, éventuellement en association avec au moins un biomarqueur choisi parmi la liste constituée : du PSA et des nucléosomes ou d'un composant de ceux-ci, dans un échantillon de sang, de sérum ou de plasma prélevé auprès du patient,
dans lequel un taux d'interleukine-8 inférieur à celui d'un témoin indique la présence d'un cancer de la prostate.

8. Procédé d'évaluation de l'aptitude d'un patient à subir une biopsie de la prostate, comprenant :
la détection ou la mesure du taux d'interleukine-8, éventuellement en association avec au moins un biomarqueur choisi parmi la liste constituée : du PSA et des nucléosomes ou d'un composant de ceux-ci, dans un échantillon de sang, de sérum ou de plasma prélevé auprès du patient ; et
dans lequel un taux d'interleukine-8 inférieur à celui d'un témoin indique que le patient nécessite une biopsie de la prostate.

9. Procédé tel que défini dans la revendication 7 ou la revendication 8, qui comprend en outre la détection ou la mesure du taux d'interleukine-6, dans lequel un taux d'interleukine-6 supérieur à celui d'un témoin indique la présence d'un cancer de la prostate ou que le patient nécessite une biopsie de la prostate.

10. Procédé tel que défini dans l'une quelconque des revendications 7 à 9, qui comprend en outre la détermination d'au moins un paramètre clinique pour le patient, de préférence dans lequel le paramètre clinique est choisi parmi : l'âge, le sexe et l'indice de masse corporelle (IMC).

11. Procédé tel que défini dans l'une quelconque des revendications 7 à 10, dans lequel l'interleukine-6 et l'interleukine-8 sont mesurées, et dans lequel la mesure combinée prend la forme d'un rapport d'interleukine-6 à interleukine-8, ou d'une différence entre celles-ci.

12. Utilisation d'un panel comprenant des réactifs pour détecter l'interleukine-8, l'interleukine-6 et les nucléosomes ou un composant de ceux-ci selon l'utilisation de l'une des revendications 1 à 6 ou de l'un quelconque des procédés des revendications 7 à 11.

13. Utilisation du panel tel que défini dans la revendication 12, qui comprend des réactifs pour détecter l'interleukine-8, l'interleukine-6, les nucléosomes ou un composant de ceux-ci et le PSA.

14. Utilisation du panel tel que défini dans la revendication 12 ou la revendication 13, dans laquelle le composant d'un nucléosome est une caractéristique épigénétique d'un nucléosome, de préférence dans laquelle la caractéristique épigénétique d'un nucléosome est l'histone H3.1.

15. Utilisation du panel tel que défini dans l'une quelconque des revendications 12 à 14, pour la détection du cancer de la prostate.
